# FASCICULE DE BREVET EUROPEEN

(11) **EP 2 430 151 B1**
(45) Date de publication et mention de la délivrance du brevet: **18.06.2014**
(21) Numéro de dépôt: 10723644.0
(22) Date de dépôt: 12.05.2010
(51) Int. Cl.: C12N 1/21, C12N 15/63, A61K 39/095

(54) **VACCIN MENINGOCOQUE A BASE DE LIPOOLIGOSACCHARIDE (LOS) PROVENANT DE SOUCHES MODIFIEES DE NEISSERIA MENINGITIDIS D'IMMUNOTYPE L6**
MENINGOKOKKENIMPFSTOFF VON LIPOOLIGOSACCHARIDEN AUS MODIFIZIERTE IMMUNOTYPE L6 NEISSERIA MENINIGITIDIS
MENINGOCOCCAL VACCINE CONSTITUTED OF LIPOOLIGOSACCHARIDES FROM MODIFIED IMMUNOTYPE L6 NEISSERIA MENINIGITIDIS

(30) Priorité: 14.05.2009 FR 0902333; 29.07.2009 US 271986 P
(43) Date de publication de la demande: 21.03.2012
(73) Titulaire: Sanofi Pasteur, 69367 Lyon Cedex 07 (FR)
(72) Inventeur: MISTRETTA, Noëlle, F-69210 Sain Bel (FR); MOREAU, Monique, F-69007 Lyon (FR); RENAULD-MONGENIE, Geneviève, F-69630 Chaponost (FR); ROKBI, Bachra, F-69003 Lyon (FR)
(86) Numéro de dépôt international: PCT/FR2010/000367
(87) Numéro de publication internationale: WO 2010/130898

(56) Documents cités:
- WO-A-2007/144316
- FR-A1- 2 692 592
- US-A1- 2006 047 106
- MACKINNON FIONA G ET AL: "Identification of a gene (Ipt-3) required for the addition of phosphoethanolamine to the lipopolysaccharide inner core of Neisseria meningitidis and its role in mediating susceptibility to bactericidal killing and opsonophagocytosis" MOLECULAR MICROBIOLOGY, vol. 43, no. 4, février 2002 (2002-02), pages 931-943, XP002602125 ISSN: 0950-382X
- KAHLER CHARLENE M ET AL: "Inner core assembly and structure of the lipooligosaccharide of Neisseria meningitidis: capacity of strain NMB to express all known immunotype epitopes" GLYCOBIOLOGY, vol. 15, no. 4, avril 2005 (2005-04), pages 409-419, XP002511441 ISSN: 0959-6658
- O'CONNOR ELLEN T ET AL: "Structural requirements for monoclonal antibody 2-1-L8 recognition of neisserial lipooligosaccharides" HYBRIDOMA, vol. 27, no. 2, avril 2008 (2008-04), pages 71-79, XP002553260 ISSN: 1554-0014
- RAM SANJAY ET AL: "Neisserial lipooligosaccharide is a target for complement component C4b. Inner core phosphoethanolamine residues define C4b linkage specificity." JOURNAL OF BIOLOGICAL CHEMISTRY, vol. 278, no. 51, 19 décembre 2003 (2003-12-19), pages 50853-50862, XP002553262 ISSN: 0021-9258
- O'CONNOR ELLEN T ET AL: "Biochemical analysis of Lpt3, a protein responsible for phosphoethanolamine addition to lipooligosaccharide of pathogenic Neisseria" JOURNAL OF BACTERIOLOGY, vol. 188, no. 3, février 2006 (2006-02), pages 1039-1048, XP002553261 ISSN: 0021-9193
- ANDERSEN SVEIN R ET AL: "Short-chain lipopolysaccharide mutants of serogroup B Neisseria meningitidis of potential value for production of outer membrane vesicle vaccines", MICROBIAL PATHOGENESIS, vol. 19, no. 3, 1995, pages 159-168, ISSN: 0882-4010

## Description

L'invention s'inscrit dans le domaine des vaccins à l'encontre des infections dues à *Neisseria meningitidis* et propose notamment une composition vaccinale comprenant deux types de lipooligosaccharide (LOS) chacun des deux types provenant d'une souche de *N. meningitidis* particulière. L'invention propose également des moyens intermédiaires contribuant à la préparation de la composition vaccinale évoquée ci-avant.

Dans le domaine des vaccins, un des enjeux majeurs des prochaines années sera notamment la mise sur le marché d'un vaccin destiné à prévenir l'ensemble des infections à *N. meningitidis* sérogroupe B. Cette bactérie est responsable d'un certain nombre de pathologies, parmi lesquelles de façon dominante, les méningites et les méningococcies, mais aussi des arthrites et péricardites. Les méningococcies peuvent se compliquer de *purpura fulminans* et de choc septique mortel.

D'une manière générale, les méningites sont soit d'origine virale, soit d'origine bactérienne. Dans les pays développés, les bactéries principalement responsables sont : *N. meningitidis* et *Streptococcus pneumoniae,* respectivement impliquées dans environ 40 et 50 % des cas de méningites bactériennes. Dans les pays en voie de développement *Haemophilus influenzae* reste aussi une source importante de méningites.

On dénombre en France, environ 600 à 800 cas par an de méningites à *N*. *meningitidis.* Aux Etats-Unis, le nombre de cas s'élève à environ 2 500 à 3 000 par an.

L'espèce *N. meningitidis* est subdivisée en sérogroupes selon la nature des polysaccharides de capsule. Bien qu'il existe une douzaine de sérogroupes, 90 % des cas de méningites sont attribuables aux sérogroupes : A, B, C, Y et W135.

Il existe des vaccins efficaces à base de polysaccharides capsulaires pour prévenir les méningites à *N. meningitidis* des sérogroupes A, C, Y et W135. Ces polysaccharides tels quels ne sont que peu ou pas immunogènes chez les enfants de moins de 2 ans et n'induisent pas de mémoire immunitaire. Toutefois, ces inconvénients peuvent être surmontés en conjuguant ces polysaccharides à une protéine porteuse.

Par contre, le polysaccharide capsulaire de *N. meningitidis* sérogroupe B n'est pas ou peu immunogène chez l'homme, qu'il soit sous forme conjuguée ou non (Bruge et al, Vaccine (2004) 22 : 1087). D'autre part, ce polysaccharide est porteur d'un épitope qui peut potentiellement réagir de manière croisée avec des tissus humains. Ainsi, il apparaît hautement souhaitable de rechercher un vaccin à l'encontre des méningites induites par *N. meningitidis* notamment du sérogroupe B autre qu'un vaccin à base de polysaccharide capsulaire.

Le lipopolysaccharide (LPS) est un constituant majeur de la membrane externe de la paroi des bactéries à Gram-négatif. Le LPS est toxique à forte dose pour les mammifères et au regard de cette activité biologique, a été dénommé endotoxine. Il est responsable du choc septique, pathologie mortelle qui se développe suite à une infection aigüe par une bactérie à Gram-négatif.

Néanmoins, le LPS n'est pas seulement toxique, il est aussi immunogène. Chez les mammifères, les anticorps anti-LPS sont générés pendant le portage et l'infection et peuvent être protecteurs. Ainsi, l'usage du LPS a déjà été envisagé dans la prophylaxie des infections dues aux bactéries à Gram-négatif et des maladies associées.

La structure du LPS est constituée d'une partie lipidique, appelée le lipide A, liée de manière covalente à une partie polysaccharidique.

Le lipide A est responsable de la toxicité du LPS. Il est fortement hydrophobe et permet d'ancrer le LPS dans la membrane externe de la paroi. Le lipide A est composé d'une structure disaccharidique substituée par des chaînes d'acides gras. Le nombre et la composition des chaînes d'acides gras varient d'une espèce à l'autre.

La partie polysaccharidique est constituée par des chaînes de carbohydrates qui sont responsables de l'antigénicité. On distingue au moins 3 grandes régions dans cette partie polysaccharidique :
(i) un core interne, constitué de monosaccharides [un ou plusieurs KDO (acide 2-céto-3-desoxy-octulosonique) et un ou plusieurs heptose (Hep)] invariants au sein d'une même espèce bactérienne ;
(ii) un core externe lié à un heptose et constitué de divers monosaccharides ; et
(iii) une chaîne externe O-spécifique constituée d'un enchaînement d'unités répétitives - unités répétitives elles-mêmes composées de un ou plusieurs monosaccharides différents.

D'une espèce à une autre, la structure du LPS est variable. C'est ainsi que par exemple, chez un certain nombre de bactéries non-entériques à Gram-négatif telles que les *Neisseriae, Bordetellae, Branhamellas, Haemophilus* et *Moraxellae,* la chaîne O-spécifique n'existe pas. La partie saccharidique de LPS de ces bactéries n'est constituée que du core oligosaccharidique. Par conséquent, le LPS de ces bactéries est souvent dénommé lipooligosaccharide (LOS).

La structure du LPS varie non seulement d'une espèce à une autre, mais peut aussi au sein d'une même espèce.

Ainsi, toutes les souches de *N. meningitidis* ne possède pas un LOS de même structure, bien que tous les LOS du méningoccoque partagent la structure de base qui est schématisée dans la formule développée suivante :

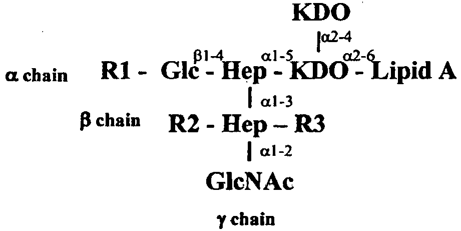

Le core externe (ou chaîne α) est variable en fonction du type d'oligosaccharide (substituant R1), accroché sur le résidu glucose porté par l'heptose I.

Alors que le lipide A est essentiellement invariant, le core interne, constitué lui aussi de manière invariante, de deux KDO (acide 2-céto, 3-desoxy, octulosonique) et de deux heptoses (HepI et HepII), porte des substitutions diverses au niveau (i) de l'heptose II (substituants R2 et R3) ; et (ii) de la chaîne γ, constituée d'une glucosamine N-acétylée (GlcNAc) qui peut être on non O-acétylée. Dans la littérature, le résidu R2 est communément appelé chaîne β, lorsque R2 est un résidu glucose.

Les souches de *N. meningitidis* sont classées en plusieurs immunotypes (IT L1 à L13), en fonction de leur réactivité avec une série d'anticorps qui reconnaissent des épitopes variés sur le LOS (Achtman et al, 1992, J. Infect. Dis. 165 : 53-68). La majorité des souches invasives à *N*. *meningitidis* sérogroupe B est d'immunotype L3,7 comme cela a été mis en évidence par la réactivité de ces souches avec un anticorps monoclonal dénommé L3,7,9. Cet anticorps monoclonal est capable de reconnaître chacun des immunotypes L3, L7 et L9 (Gu et al, J. Clin. Microbiol. (1992) 30 : 2047 ; Moran et al, Infect. Immun. (1994) 62 (12): 5290 ; et Scholten et al, J. Med. Microbiol. (1994) 41 : 236). Le LOS de ces souches est répertorié de même en 13 immunotypes (IT L1 à L13). Les différences entre immunotypes proviennent de variations dans la composition et dans la conformation des chaînes oligosaccharidiques. Ceci transparaît dans le tableau ci-dessous (tableau I), dérivé du tableau 2 de Braun et al, Vaccine (2004) 22 : 898, complété par des données obtenues ultérieurement et relatives aux immunotypes L9 (Choudhury et al, Carbohydr. Res. (2008) 343 : 2771) et L11 (Mistretta et al, (2008) Poster at the 16t International Pathogenic Neisseria Conference, Rotterdam):

**Tableau I :**

| **IT** | **R1 (chaîne α)** | **R2** | **R3** |
|---|---|---|---|
| **L1** | NeuNAcα2-6 Galα1-4 Galβ1-4 | PEA-3 | - |
| **L2** | NeuNAcα2-3 Galβ1-4 GlcNAcβ1-3 Galβ1-4 | Glcα (1-3) | PEA-6 ou PEA-7 |
| **L3** | NeuNAcα2-3 Galβ1-4 GlcNAcβ1-3 Galβ1-4 | PEA-3 | - |
| **L4** | NeuNAcα2-3 Galβ1-4 GlcNAcβ1-3 Galβ1-4 | - | PEA-6 |
| **L5** | NeuNAcα2-3 Galβ1-4 GlcNAcβ1-3 Galβ1-4 Glcβ1-4 | Glcα (1-3) | - |
| **L6** | GlcNAcβ1-3 Galβ1-4 | - | PEA-6 ou PEA-7 |
| **L7** | Galβ1-4 GlcNAcβ1-3 Galβ1-4 | PEA-3 | - |
| **L8** | Galβ1-4 | PEA-3 | - |
| **L9** | Galβ1-4 GlcNAcβ1-3 Galβ1-4 | - | PEA-6 |
| **L10** | n.d. | n.d. | n.d. |
| **L11** | Glcβ1-4 | PEA-3 | PEA-6. |
| **L12** | n.d | n.d. | n.d. |
| **L13** | n.d | n.d. | n.d. |

| | | | |
|---|---|---|---|
| n.d. : non déterminé. | | | |

Entre autre, on peut noter que certains LOS peuvent être sialylés (présence d'acide N-acétyl neuraminique sur le résidu galactose (Gal) terminal de la chaîne α). Ainsi, les immunotypes L3 et L7 ne diffèrent que par la présence / absence respective de cette sialylation. Par ailleurs, la plupart des LOS sont substitués par un groupement O-acétyle sur le résidu glucosamine (α-GlcNAc) du core interne (Wakarchuk et al. (1998) Eur. J. Biochem. 254 : 626 ; Gamian et al. (1992) J. Biol. Chem. 267 : 922 ; Kogan et al (1997) Carbohydr. Res. 298 : 191 ; Di Fabio et al. (1990) Can. J. Chem. 68 : 1029 ; Michon et al. (1990) J. Biol. Chem. 275 : 9716 ; Choudhury et al. (supra) ; et Mistretta et al. (supra).

Les autres variations dans la structure du LOS sont dues à différents facteurs génétiques au nombre desquels :
(i) la présence / absence de certains gènes impliqués dans la voie de biosynthèse du LOS et aux associations possibles des gènes entre eux ;
(ii) la variation de phase à laquelle sont soumis certains gènes ;
(iii) la recombinaison homologue [certains gènes possédant des régions conservées (*lgt*B, *lgt*E et *lgt*H) et d'autres gènes étant des hybrides (*lgt*Z est l'hybride des gènes *lgt*A et *lgt*B)*,* des réarrangements peuvent se produire] ; et
(iv) les mutations.

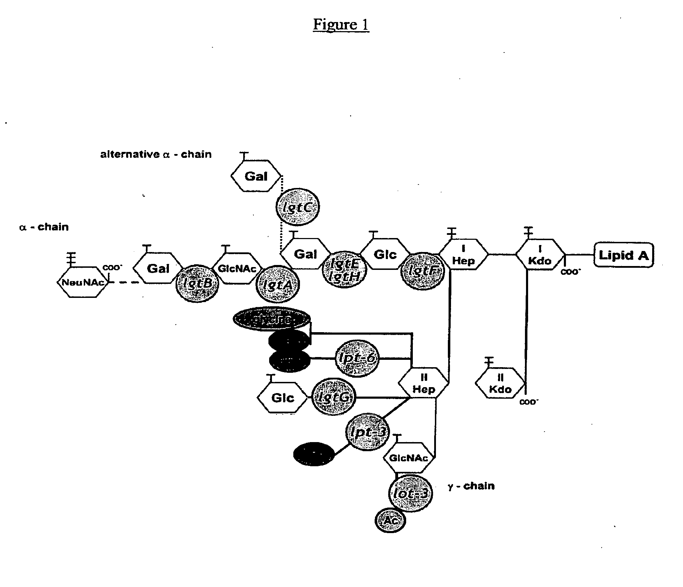

Les gènes impliqués dans la voie de biosynthèse du LOS (à l'exception de deux) sont répartis en trois loci (*lgt*-1, *lgt*-2 et *lgt*-3). La description de ces gènes ainsi que leur fonction est fournie ci-après, illustrée de manière schématique par la figure 1 qui présente la structure du LOS de N. *meningitidis,* les différents sites où la variabilité s'exprime ainsi que les niveaux d'intervention des gènes.

Les gènes hors locus sont *lpt*3 et *lot*3. Le gène *lpt*3 code pour une PEA transférase. Cette enzyme a la capacité d'accrocher un résidu phosphoéthanolamine (PEA) en position O-3 de l'heptose II. Mackinnon et al, Mol. Microbiol. (2002) 43 (4): 931 est l'article scientifique qui identifie le gene *lpt*3 comme étant celui qui permet l'addition d'un groupement PEA en position 03. Dans le cadre des travaux d'identification reportés, la souche *Nm* NGH15 qui ne réagit pas avec le monoclonal B5 connu pour cibler le groupement PEA 03 est modifiée par voie recombinante pour exprimer le gène *lpt*3 de la souche MC58. La chaîne α du LOS de la souche NGH15 est du type L7 / L9.

Le gène *lot*3 code pour une LOS O-acétyltransférase qui possède la capacité de O-acétyler la chaîne γ. Il est soumis à une variation de phase.

Le locus *lgt*-1 comporte 7 gènes : *lgt*A*, lgt*B, *lgt*C, *lgt*D, *lgt*E, *lgt*H et lgtZ codant chacun pour une glycosyl transférase particulière. Parmi ces gènes, *lgt*A et *lgt*C sont soumis à une variation de phase. *lgt*E et *lgt*H présentent une variation allélique : le codon déterminant l'acide aminé en position 153 code soit pour un résidu thréonine (et dans ce cas l'enzyme qui en résulte est une Gal-transférase) ; soit pour un résidu méthionine (et dans ce cas l'enzyme qui en résulte est une Glc-transférase).

Le locus *lgt*-1 est classé en 8 types génétiques (Zhu et al, Microbiology (2002) 148 : 1833).

Le locus *lgt*-2 comporte 2 gènes : *lgt*F et *lgt*K codant pour des glycosylases. Le produit du gène *lgt*F intervient dans la construction de la chaîne α en permettant la fixation du résidu glucose sur l'heptose 1 et donc n'intervient pas sur la nature de l'immunotype ; ni le gène *lgt*K d'ailleurs.

Le locus *lgt*-3 comporte 2 gènes : *lgt*G et *lpt*6*.* Le gène *lgt*G code pour une Glc synthétase qui possède la capacité d'accrocher un résidu glucose en position O-3 de l'heptose II. Le gène *lpt6* code pour une PEA transférase qui possède la capacité d'accrocher un substituant phosphoéthanolamine (PEA) en position O-6 ou O-7 de l'heptose II. Le gène *lgt*G est soumis à une variation de phase. Lorsqu'il est « On » et accompagné d'un gène *lpt*3 fonctionnel, l'accrochage du résidu glucose se fait toujours au dépend du PEA (dont l'accrochage est médié par *lot*3).

Le locus *lgt*-3 est classé en 5 types génétiques (Wright et al, J. Bact. (Oct. 2004) : 6970).

Le motif de carbohydrates Galβ1-4GlcNAcβ1-3Galβ1-4Glcβ1-4 ou motif lacto-N-néotétraose qui est présent dans la chaîne α de certains immunotypes du LOS de *N*. *meningitidis,* constitue un épitope qui peut potentiellement réagir de manière croisée avec les érythrocytes humains. Ainsi, en vue de fabriquer un vaccin destiné aux humains, il convient de choisir un LOS ne possédant pas ce motif. US 2006/0047106 décrit des structures inner core du LOS de *Nm* pouvant être utilisées en prophylaxie. Ces structures sont porteuses sur l'heptose 1, d'un groupe R1 qui peut être un mono ou di-glucose. Ainsi, aucune de ces structures ne possède de motif lacto-N-néotétraose. Elles ne possèdent pas non plus de chaîne α de type L6.

Par contraste avec US 2006/0047106, il peut donc être particulièrement avantageux d'utiliser un LOS provenant de souches d'immunotype L6 ou L8.

Néanmoins, une étude de génotypage de souches épidémiologiques a permis de trouver que les souches d'immunotype L6 ou L8 devaient être optimisées afin de modifier la structure de leur LOS d'origine ; et cela en vue de fabriquer un vaccin amélioré à base de LOS.

L'étude de génotypage en question à été faite en deux temps.

Une vingtaine de souches de *N. meningitidis* ont tout d'abord été analysées à la fois par génotypage et par analyse biochimique (spectrométrie de masse et résonance magnétique nucléaire). Dans un premier temps, l'immunotype de ces souches a été prédit à partir des résultats du génotypage. Les résultats de l'analyse biochimique ont mis en évidence une excellente corrélation entre la structure effectivement déterminée et l'immunotype prédit par génotypage. Compte tenu de l'étroit parallélisme entre les résultats du génotypage et ceux de l'analyse biochimique, il a été possible dans un deuxième temps, de poursuivre valablement l'analyse d'une collection de souches beaucoup plus étendue, uniquement par génotypage.

Ainsi, on a rassemblé une collection de 163 souches dont la plupart ont été fournies par les laboratoires des Drs D.A. Caugant (NIPH, Oslo, Norvège), D. Martin (ESR, Porirua, Nouvelle-Zélande) et M. Diggle (SMPRL, Glasgow, Royaume-Uni). Les souches de cette collection proviennent du monde entier. Environ la moitié d'entre elles ont été isolées en Europe. Elles sont en très large majorité de sérogroupe B et sont réparties dans les 6 complexes épidémiologiques majeurs retrouvés en Europe chez les souches invasives de sérogroupe B : soient les complexes MLST (multilocus sequence type) ST-8, ST-11, ST-18, ST 32, ST-41/44 et ST-269.

En effet, en matière d'épidémiologie, les données européennes les plus récentes indiquent que 64 % des souches invasives de méningo B se répartissent dans ces 6 complexes alors qu'à ce jour 50 complexes ont été décrits (19 % des souches européennes n'ayant pu être assignées à un complexe déterminé).

Le tableau qui suit (Tableau II) fournit plus de détails concernant ces 163 souches et indique entre parenthèses, les précédentes dénominations ou noms des complexes MLEE (multi electrophoretic enzyme) ou complexes électrophorétiques (ET) correspondants :

**Tableau II :**

| Complexe | Nombre de souches | Origine (nombre de pays) | Période d'isolation | Source principale |
|---|---|---|---|---|
| ST-41/44 (linéage III) | 34 | 9 | 1963-1994 | D. Caugant / D. Martin |
| ST-32 (ET-5) | 53 | 10 | 1981-1996 | D. Caugant |
| ST-269 | 14 | 2 | 1988-2007 | D. Caugant |
| ST-18 | 7 | 2 | 1985-2005 | D. Caugant |
| ST-8 (cluster A4) | 28 | 12 | 1967-1994 | D. Caugant / M. Diggle |
| ST-11 (ET-37) | 27 | 7 | 1969-1988 | D. Caugant / M. Diggle |

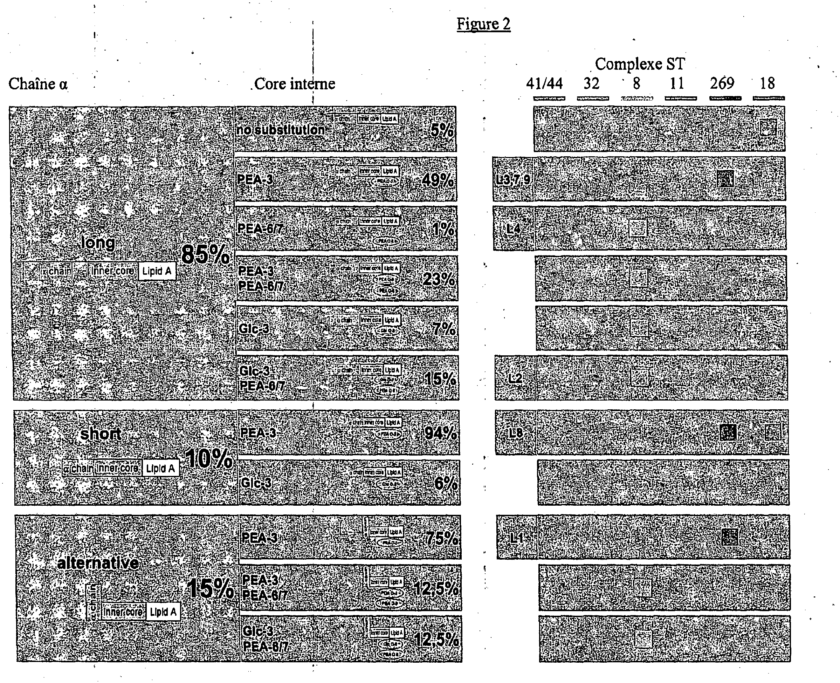

Les gènes participant à la biosynthèse du LPS qui ont été analysés par génotypage sont les suivants : *lgt*A*, lgt*B*, lgt*C, *lgt*E et *lgt*H; *lgt*F; *lgt*G et *lpt6 ; lpt*3 et *lot*3. Cette analyse a permis de prédire la structure du LOS dans les 6 complexes épidémiologiques majeurs du méningoccoque B. Les résultats sont présentés dans la figure 2. On notera que les résultats du génotypage de certaines souches sont tels qu'il faille en déduire que telle souche est capable de fabriquer plusieurs types de LOS. Le LOS d'une telle souche peut donc être classé dans plusieurs catégories. *In fine,* ceci explique par exemple qu'une souche puisse être comptabilisée dans plusieurs catégories de chaîne *α*.

Les résultats du génotypage portant sur 3 des 4 les gènes intervenant dans la biosynthèse du core interne et agissant sur sa variabilité (soient *lgt*G, *lpt*6 et *lpt*3) ainsi que la structure qui en est déduite sont présentés en détails dans le tableau III suivant :

**Tableau III : Association entre complexes clonaux et phénotype / génotype de l'inner core**

| | | | | **Complexes ST** | | | | | | **Somme des souches** | |
|---|---|---|---|---|---|---|---|---|---|---|---|
| | | | | **41/44** | **32** | **269** | **18** | **8** | **11** | | |
| | | | | (lineage III) | (ET-5) | | (clusterA4) | (cluster A4) | (ET-37) | | |
| *Distribution des 6 complexes épidémiologiques majeurs chez les souches invasives de sérogroupe B* | | | | 25 % | 20 % | 7% | 4% | 4% | 4% | 64 % | |
| Pas de Substitution | *lpt*3- | *lgt*G- | *lpt6-* | | | | 6 | | | 7 | Pas de Substitution |
| | *Ipt*3+ délété | *lgt*G Off | *lpt6-* | 1 | | | | | | 4,3% | |
| | *lpt*3- | | | | | | | | | | |
| Glu-3 | *lpt*3- | *lgt*G On | *lpt*6- | | | | | | | 10 | Glu-3 |
| | *lpt*3+ | *lgt*G On | *lpt*6- | | 9 | | | 1 | | 6,13 % | |
| PEA-3 | *lpt*3+ | *lgt*G- | *lpt*6- | 33 | | 14 | | | | 92 | PEA-3 |
| | *lpt*3+ | *lgt*G Off | *lpt*6- | | 44 | | 1 | | | 56,44 % | |
| PEA-3 | *lpt*3+ | *lgt*G- | *lpt*6+ | | | | | | | 32 | PEA-3 |
| PEA-6 | *lpt*3+ | *lgt*G Off | *lpt*6+ | | | | | 13 | 19 | 19,63 % | PEA-6 |
| Glu-3 | *lpt3-* | *lgt*G On | *lpt*6+ | | | | | | | 21 | Glu-3 |
| PEA-6 | *lpt*3+ | *lgt*G On | *lpt*6+ | | | | | 13 | 8 | 12,88 % | PEA-6 |
| PEA-6 | *lpt3*- | *lgt*G*-* | *lpt*6+ | | | | | | | 1 | PEA-6 |
| | *lpt3-* | *lgt*G Off | *lpt*6+ | | | | | 1 | | 0,60% | |
| Nombre de souches testées | | | | 34 | 53 | 14 | 7 | 28 | 27 | 163 | |

D'autre part, l'étude de génotypage portant sur le gène *lot*3, révèle que la très grande majorité des souches testées O-acétylent leur LOS (gène *lot3* présent et « On »). En cohérence avec les résultats de l'étude de génotypage portant sur les gènes *lgt*G, *lpt6* et *lpt*3, on propose une composition vaccinale destinée à prévenir ou traiter les infections dues à *N. meningitidis* qui comprend un ou plusieurs LOS de *N. meningitidis* ; ceci afin de (i) traiter ou prévenir au moins 60 %, avantageusement au moins 70 %, de préférence au moins 80 % des infections dues à *N. meningitidis,* notamment de sérogroupe B ou (ii) traiter ou prévenir des infections dues au moins à 60 %, avantageusement au moins 70 % de préférence au moins 80 % des souches de *N. meningitidis,* notamment de sérogroupe B.

A cette fin, le choix d'un LOS possédant une chaîne α de type L6 et un résidu heptose II du core interne substitué en position O-3 par un résidu phosphoéthanolamine, s'avère particulièrement intéressant. Or, comme reporté précédemment dans le tableau I ci-dessus, les souches d'immunotype L6 sont porteuses d'un unique substituant PEA en position 6/7.

Ainsi, lorsque la composition vaccinale ne comporte qu'un seul LOS, ce dernier doit avoir été nécessairement optimisé. Lorsque la composition vaccinale comporte au moins deux LOS, un des au moins deux LOS doit avoir été nécessairement optimisé ; le deuxième pouvant être un LOS naturel (non-optimisé).

Afin de parvenir à fabriquer un vaccin selon l'invention, on propose tout d'abord trois procédés de fabrication de souche énoncés comme suit :
(i) Un procédé de fabrication d'une souche de *N. meningitidis* possédant un lipooligosaccharide (LOS) constitué notamment d'un lipide A, d'un core interne, d'une chaîne α de type L6, dans lequel le résidu heptose II du core interne porte en position O-3 et en position O-6 ou O-7, un substituant phosphoéthanolamine (PEA) ; procédé selon lequel on modifie une souche de *N. meningitidis* d'immunotype L6 de telle sorte qu'elle exprime un gène *lpt*3 ;
(ii) Un procédé de fabrication d'une souche de *N. meningitidis* possédant un lipooligosaccharide (LOS) constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; procédé selon lequel on modifie une souche de *N. meningitidis* d'immunotype L6 de telle sorte qu'elle exprime un gène *lpt*3 et qu'elle n'exprime plus le gène *lpt*6 ; et
(iii) Un procédé de fabrication d'une souche de *N. meningitidis* possédant un lipooligosaccharide (LOS) constitué notamment d'un lipide A, d'un core interne, d'une chaîne α de type L8, dans lequel le résidu heptose II du core interne porte en position O-3 et en position O-6 ou O-7 un substituant phosphoéthanolamine (PEA) ; procédé selon lequel on modifie une souche de *N. meningitidis* d'immunotype L8 de telle sorte qu'elle exprime un gène *lpt*6.

Le sérogroupe de *N. meningitidis* contre lequel il est impératif de proposer un vaccin en priorité est le sérogroupe B (des vaccins contre les autres sérogroupes prévalents A, C, Y et W135 sont déjà disponibles). Or la purification du LOS à partir d'une souche de sérogroupe B peut conduire à un produit contenant de la capsule B résiduelle indésirable dans le vaccin. Pour pallier ces difficultés, on a maintenant trouvé qu'un LOS *adhoc* issu d'une souche de sérogroupe A pouvait remplir les besoins en matière de vaccination contre le sérogroupe B. C'est pourquoi dans les procédés de fabrication (i) et (ii) selon l'invention, on utilise de préférence comme souche de départ une souche de *N. meningitidis* d'immunotype L6, sérogroupe A.

Une souche pouvant être utilisée dans les procédés de fabrication peut exprimer un LOS possédant un lipide A non détoxifié. En particulier, le gène *msb*B peut être fonctionnel.

Une souche de ce type est la souche C708 déposée le 11 mars 2008, auprès de la Collection Nationale de Culture de Microorganisme, 25 rue du Dr Roux 75015 Paris, selon les termes du traité de Budapest. Cette souche porte le numéro d'ordre CNCM I-3942. Cette souche possède entre autre un gène *lgt*A actif (gène allumé « ON ») ; un gène *lgt*B *-* (gène non fonctionnel); un gène *lgt*G éteint (« Off ») ; un gène *lpt*3 tronqué ; un gène *lpt*6 actif ; un gène *lot3* actif ; et un gène msbB actif.

La souche C708 comporte un gène *lpt*3 tronqué. Pour la modifier de telle sorte que le LOS soit porteur d'un substituant PEA en position 03, on peut restaurer la fonctionnalité du gène *lpt*3, notamment par recombinaison homologue faisant usage d'un gène *lpt*3 complet (full-length). Lorsque cette souche est utilisée dans le procédé (ii) selon l'invention, il convient en plus de désactiver le gène *lpt*6 pour obtenir une souche qui n'exprime plus ce gène. La désactivation de ce gène peut être notamment réalisée par délétion totale ou partielle du gène *lpt6* ou bien encore par insertion intra-génique d'une séquence non pertinente, par exemple d'un gène de résistance à un antibiotique.

Sous un autre aspect, l'invention a aussi pour objet :
(i) Une souche de *N. meningitidis,* notamment de sérogroupe A, possédant un lipooligosaccharide (LOS) constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ;
(ii) Une souche de *N. meningitidis* de sérogroupe A, qui possède un lipooligosaccharide (LOS) constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ;
(iii) Un lipooligosaccharide (LOS) de *N. meningitidis* constitué notamment d'un lipide A qui peut être détoxifié ou non, d'un core interne, d'une chaîne α de type L6, dans lequel le résidu heptose II du core interne porte en position O-3 et en position O-6 ou O-7 un substituant phosphoéthanolamine (PEA) ; et
(iv) Un lipooligosaccharide (LOS) de *N. meningitidis* constitué notamment d'un lipide A non-détoxifié, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7.

De manière avantageuse, les souches selon l'invention possèdent un gène *lot3* fonctionnel, en variation de phase « On » de manière à ce que le LOS qu'elles produisent soit O-acétylé, au moins de manière partielle, au niveau de leur chaîne γ. Les souches selon l'invention peuvent posséder un gène *msb*B fonctionnel et/ou exprimer un LOS notamment constitué d'un lipide A non-détoxifié.

De manière avantageuse, un LOS selon l'invention possède une chaîne γ qui est O-acétylée, au moins de manière partielle.

Un LOS entrant dans la composition d'un vaccin selon l'invention, peut être préparé selon l'un des trois procédés suivants :
(i) Un procédé de préparation d'un lipooligosaccharide (LOS) de *N. meningitidis* constitué notamment d'un lipide A, d'un core interne, d'une chaîne α de type L6, dans lequel le résidu heptose II du core interne porte en position O-3 et en position O-6 ou O-7, un substituant phosphoéthanolamine (PEA) ; procédé selon lequel (a) on cultive une souche obtenue à partir du procédé de fabrication (i) selon l'invention ou la souche (i) selon l'invention et (b) on récolte le LOS à partir de la culture obtenue à l'étape (a) ;
(ii) Un procédé de préparation d'un lipooligosaccharide (LOS) de *N. meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; procédé selon lequel (a) on cultive une souche obtenue à partir du procédé de fabrication (ii) ou la souche (ii) selon l'invention et (b) on récolte le LOS à partir de la culture obtenue à l'étape (a) ; et
(iii) Un procédé de préparation d'un lipooligosaccharide (LOS) de *N. meningitidis* constitué notamment d'un lipide A, d'un core interne, d'une chaîne α de type L8, dans lequel le résidu heptose II du core interne porte en position O-3 et en position O-6 ou O-7, un substituant phosphoéthanolamine (PEA) ; procédé selon lequel (a) on cultive une souche obtenue à partir du procédé de fabrication (iii) et (b) on récolte le LOS à partir de la culture obtenue à l'étape (a).

Dans chacun des trois procédés de préparations, le LOS peut être récolté soit sous forme associée à des vésicules de membrane externe (OMVs) ; soit extrait pour être purifié par la suite.

Le premier cas revient à récolter des OMVs qui contiennent du LOS. De telles OMVs peuvent être isolées selon de nombreuses techniques. Voir par exemple WO 04/014417, Fredriksen et al, NIPH Annals (1991) 14 : 67-79 ; Zollinger et al, J. Clin Invest (1979) 63 : 836-848 ; Saunders et al, Infect. Immun. (1999) 67: 113-119 ; et Drabick et al, Vaccine (1999) 18 : 160-172. Ces techniques peuvent être divisées en deux grands groupes ; celles qui font usage du desoxycholate (DOC) à une concentration de l'ordre de 0.5 % ; et celles qui utilisent des concentrations moindre ou pas de DOC du tout. Les techniques qui se passent de DOC présentent l'avantage de concentrer le LOS dans les OMVs (environ 10 fois plus de LOS que dans les OMVs extraites par une technique au DOC). Toutefois, les techniques faisant usage de faibles doses de DOC peuvent aussi être avantageuses en ce qu'elles peuvent réduire la toxicité résiduelle du LOS tout en permettant d'obtenir des taux de concentration en LOS intéressants. Les OMVs une fois extraites peuvent être purifiées par des techniques d'ultrafiltration ou de diafiltration connues de l'homme du métier et décrites par exemple dans Frasch et al. "Outer membrane protein vesicle vaccines for meningococcal disease" in Methods in Molecular Medicine, vol 66, Meningococcal Vaccines : Methods and Protocols (2001) : 81-107 (Edited by AJ. Pollard and M.C. Maiden, Humana Press Totowa, NJ).

Dans le deuxième cas, le LOS est extrait à partir d'une culture bactérienne puis purifié selon des méthodes classiques. De nombreux procédés de purification sont décrits dans la littérature. A titre d'exemple, on cite Gu & Tsaï, 1993, Infect. Immun. 61 (5) : 1873, Wu et al, 1987, Anal. Biochem. 160: 281 et US 6,531,131. Une préparation de LPS peut être aussi quantifiée en mettant en oeuvre des techniques connues. Une méthode pratique consiste à doser le KDO par chromatographie HPAEC- PAD (high performance anion exchange chromatography).

### Détoxification du LOS

Pour incorporation dans un vaccin, le LOS se doit d'être détoxifié. La toxicité du LOS est due à son lipide A. Néanmoins, il n'est pas impératif de retirer le lipide A dans son intégralité ; ni de le modifier par exemple par mutation (e.g. mutation *msb*B minus). En effet, la toxicité étant plus particulièrement liée à une conformation supra moléculaire conférée par la totalité des chaînes d'acides gras portées par le noyau disaccharidique du lipide, selon un mode avantageux, il suffit d'agir au niveau de ces chaînes.

La détoxification peut être obtenue selon des approches diverses : chimique, enzymatique, génétique ou bien encore par complexation avec un peptide analogue de la polymixine B ou bien encore par incorporation / formulation en liposomes.

Le niveau de détoxification du LOS, peut être apprécié *i.a.* selon l'un des deux tests standard suivants :
- Le test pyrogène chez le lapin. Ce test, les calculs et leur lecture et ont été réalisés selon les principes énoncés par la Pharmacopée Européenne (Edition 6.0, paragraphe 2.6.8.).
- Le test LAL (Limulus Amebocyte Lysate) réalisé selon les principes énoncés par la Pharmacopée Européenne (Edition 6.0, paragraphe 2.6.14.).

### Détoxification par voie chimique

L'approche chimique consiste à traiter le LOS par un agent chimique. Selon un mode particulier, le LOS est soumis à une hydrolyse acide douce avec de l'acide acétique qui élimine le lipide A ainsi que les ou les KDO en ramification quand celui-ci (ceux-ci) est (sont) présent(s) dans la structure du LOS. Un tel traitement est par exemple décrit dans Gu & Tsai Infect. Immun. (1993) 61 : 1873. Selon un mode alternatif et préféré, le LOS est soumis à une dé-O-acylation, de préférence primaire, *i.a.* par traitement à l'hydrazine qui hydrolyse les chaînes d'acides gras primaires estérifiées du lipide A. Un tel traitement est par exemple décrit dans USP 6,531,131, Gupta et al, Infect. Immun. (1992) 60 (8) : 3201 et Gu et al, Infect. Immun. (1996) 64 (10) : 4047. *Détoxification par voie enzymatique*

L'approche enzymatique consiste à mettre le LOS en présence de lipases capables de digérer les chaînes d'acides gras esterifiées du lipide A. De telles lipases sont produites par l'amibe *Dictyostelium discoideum.* Selon un mode particulièrement avantageux, on cultive ensemble (co-culture) l'amibe et une bactérie à Gram-négatif pouvant être phagocytée par l'amibe, telle que *N. meningitidis.* Puis on récupère le surnageant et on extrait le LOS du surnageant qui est alors dépourvu des chaînes d'acides gras. Ce peut être également une acyloxyacyl hydrolase produite par certaines cellules humaines (brevet WO 87/07297 Munford R.) ou par *Salmonella typhimurium* (Trent et al 2001 J. Biol. Chem. 276 : 9083-9092 ; Reynolds et al. 2006 J. Biol. Chem. 281 : 21974-21987) (enzyme codée par les gènes *PagL* ou *LpxR* dans ce dernier cas).

### Détoxification par voie génétique

L'approche génétique consiste à utiliser un LOS produit par une souche bactérienne dont le génotype est tel que l'entité du LOS normalement responsable de sa toxicité (le lipide A et plus particulièrement les queues lipidiques du lipide A) présente un degré de toxicité largement réduit ou même inexistant. Une telle souche bactérienne peut être commodément obtenue par mutation. A partir d'une souche sauvage (c'est-à-dire produisant un LOS toxique), il s'agit alors d'inactiver par mutation certains gènes intervenant dans la biosynthèse des chaînes d'acides gras ou dans leur accrochage sur le noyau disaccharidique du lipide A. Ainsi, on peut prévoir d'inactiver les gènes *lpx*L1 ou *lpx*L2 (aussi appelés *htr*B1 / *htr*B2) de *N. meningitidis* ou leur équivalents dans d'autres espèces (par exemple, les équivalent des gènes *lpx*L1 et *lpx*L2 du méningoccoque sont appelés respectivement appelés *msb*B ou *lpx*M et *htr*B ou *lpx*L chez *E. coli.*). Une mutation inactivant l'un de ces gènes conduit à un LOS dépourvu d'une ou de deux chaînes acyles secondaires. Des mutants *lpx*L1 ou L2 de *N. meningitidis* ou *d'Haemophilus influenzae* sont en particulier décrits dans les demandes de brevet WO 00/26384, US 2004/0171133 et WO 97/019688. Chez *N. meningitidis* le gène endogène *lpxA* peut être aussi remplacé par le gène homologue en provenance d'*E*. *coli* ou *Pseudomonas aeruginosa.* Les chaînes d'acides gras en sont modifiées, avec pour conséquence un lipide A moins toxique (Steeghs et al, Cell. Microbiol. (2002) 4 (9) : 599). L'approche génétique est privilégiée lorsque le LOS est purifié sous forme d'OMVs.

### LOS détoxifié par complexation avec un peptide analogue de la polymixine B

Une quatrième approche consiste à complexer le LOS avec un peptide analogue de la polymixine B, comme cela est par exemple décrit dans la demande de brevet WO 06/108586. Le LOS complexé et par conséquent, détoxifié est appelé endotoxoïde.

L'analogue de la polymixine B entrant dans la composition d'un endotoxoïde utile aux fins de la présente invention peut être n'importe quel peptide capable de détoxifier le LPS par simple complexation. De tels peptides sont notamment décrits dans les brevets ou demandes de brevet US 6,951,652, EP 976 402 et WO 06/ 06/108586.

Ainsi un peptide avantageux peut être le peptide de formule (I) NH₂-A-Cysl-B-Cys2-C-COOH, dans laquelle :
- A est un peptide de 2 à 5, de préférence 3 ou 4 résidus d'acide aminé, dans lesquels au moins 2 résidus d'acide aminé, sont indépendamment choisis parmi Lys, Hyl (hydroxylysine), Arg et His ;
- B est un peptide de 3 à 7, de préférence 4 ou 5 résidus d'acide aminé, qui comporte au moins deux, de préférence trois résidus d'acide aminé choisis parmi Val, Leu, Ile, Phe, Tyr et Trp ; et
- C est facultatif (cette position peut être vide ou pas) et est un résidu d'acide aminé ou un peptide constitué de 2 à 3 résidus d'acide aminé ;
à condition que rapport acide aminé cationique / acide aminé hydrophobe dans le peptide de formule I soit de 0.4 à 2, avantageusement de 0.5 à 1.2 ou 1.5, de préférence de 0.6 à 1 ; mieux de 0.6 à 0.8 ; par exemple de 0.75.

De préférence dans le peptide de formule (I) la position C est une position vide.

Des exemples particuliers du peptide de formule (I) sont les peptides suivants :
NH₂-Lys-Thr-Lys-Cysl-Lys-Phe-Leu-Lys-Lys-Cys2-COOH (peptide SAEP2) ;
NH₂-Lys-Thr-Lys-Cysl-Lys-Phe-Leu-Leu-Leu-Cys2-COOH (peptide SAEP2-L2) ;
NH₂-Lys-Arg-His-Hyl-Cys1-Lys-Arg-Ile-Val-Leu-Cys2-COOH ;
NH₂-Lys-Arg-His-Cysl-Val-Leu-Ile-Trp-Tyr-Phe-Cys2-COOH ;
NH₂-Lys-Thr-Lys-Cysl-Lys-Phe-Leu-Leu-Leu-Cys2-COOH ; et
NH₂-Hyl-Arg-His-Lys-Cysl-Phe-Tyr-Trp-Val-Ile-Leu-Cys2-COOH.

Les peptides de formule (I) peuvent être sous forme de monomère ou de préférence sous forme de dimère, parallèle ou anti-parallèle.

D'une manière générale, on peut aussi employer un peptide dimérique de formule (II) 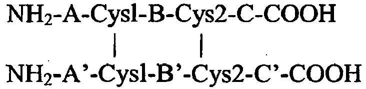 dans laquelle les deux résidus de Cysl sont liés ensemble par un pont disulfure et les deux résidus Cys2 sont liés ensemble par un pont disulfure ; ou de formule (III) 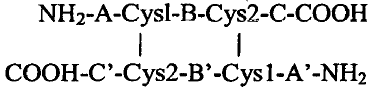 dans laquelle les résidus Cysl sont liés aux résidus Cys2 par des ponts disulfure inter-chaîne peptidique ; formules (II) et (III) dans lesquelles :
- A et A' sont de manière indépendante un peptide de 2 à 5, de préférence 3 ou 4 résidus d'acide aminé, dans lesquels au moins 2 résidus d'acide aminé, sont indépendamment choisis parmi Lys, Hyl (hydroxylysine), Arg et His ;
- B et B' sont de manière indépendante un peptide de 3 à 7, de préférence 4 ou 5 résidus d'acide aminé, qui comportent au moins deux, de préférence trois résidus d'acide aminé ont indépendamment choisi parmi Val, Leu, Ile, Phe, Tyr et Trp ; et
- C et C' sont facultatifs (ces positions peuvent être vides ou pas) et sont de manière indépendante un résidu d'acide aminé ou un peptide de 2 à 3 résidus d'acide aminé ;
à condition que le rapport acide aminé cationique / acide aminé hydrophobe dans le dimère de formule (II) ou (III), soit de 0.4 à 2, avantageusement de 0.5 à 1.2 ou 1.5, de préférence de 0.6 à 1 ; mieux de 0.6 à 0.8 ; par exemple. 0.75.

Avantageusement, A et A' sont de manière indépendante un peptide de 2 à 5, de préférence 3 ou 4 résidus d'acide aminé, dans lesquels au moins un, de préférence 2 résidus d'acide aminé, sont choisis de manière indépendante parmi Lys, Hyl, Arg et His ; et le cas échéant, ceux qui ne sont pas choisis parmi Lys, Hyl, Arg et His (« les résidus restants d'acide aminé »), étant choisis dans le groupe des résidus d'acide aminé non-chargés, polaires ou non-polaires ; de préférence Thr, Ser et Gly ; de manière tout particulièrement préférée Thr.

Quand A et A' comportent 3 résidus d'acide aminé, chacun d'entre eux peut être un résidu cationique ; ou alternativement, deux sur trois résidus sont les acides aminés cationiques, tandis que le résidu restant est choisi dans le groupe des résidus d'acide aminé non-chargés, polaires ou non-polaires ; de préférence Thr, Ser et GIy ; de manière tout particulièrement préférée Thr.

Quand A et A' comportent 4 résidus d'acide aminé, on préfère que deux ou trois sur quatre résidus soient choisis parmi les groupes de résidus d'acide aminé cationiques comme défini ci-dessus, tandis que le résidu restant (s) est (sont) choisi(s) dans le groupe des résidus des résidus d'acide aminés non-chargés polaires ou non polaires comme défini ci-dessus.

Quand A et A' comportent 5 résidus d'acide aminé, on préfère que trois ou quatre sur cinq résidus soient choisis parmi les groupes de résidus cationiques d'acide aminé comme défini ci-dessus, tandis que le résidu restant (s) est (sont) choisi(s) dans le groupe des résidus des résidus d'acide aminés non-chargés polaires ou non polaires comme défini ci-dessus.

Avantageusement, B et B' sont de manière indépendante un peptide de 3 à 7, de préférence 4 ou 5 résidus d'acide aminé, qui comporte au moins deux, de préférence trois résidus d'acide aminé indépendamment choisis parmi Val, Leu, Ile, Phe, Tyr et Trp ; de préférence Leu, Ile et Phe ; et le cas échéant, ceux qui ne sont pas choisis parmi Val, Leu, Ile, Phe, Tyr et Trp (« les résidus restants d'acide aminé »), étant indépendamment choisis dans le groupe constitué de Lys, Hyl, Arg et His. Comme on peut facilement le comprendre, B et B' peuvent comporter jusqu'à 7 résidus d'acide aminé choisis de manière indépendante parmi Val, Leu, Ile, Phe, Tyr et Trp.

Avantageusement, B et B' comportent la séquence - X1 - X2 - X3 -, dans lequel X1 et X2 ; X2 et X3 ; ou X1, X2 et X3 sont indépendamment choisis parmi Val, Leu, Ile, Phe, Tyr et Trp ; de préférence parmi Leu, Ile et Phe. Dans un mode de réalisation préféré, la séquence - X1 - X2 - X3 - comporte le motif Phe-Leu.

Les modes de réalisation particuliers de B et B' incluent :
(i) la séquence - X1 - X2 - X3 - dans laquelle :
   X1 est Lys, Hyl, His ou Arg, de préférence Lys ou Arg ; de préférence Lys ;
   X2 est Phe, Leu, Ile, Tyr, Trp ou Val ; de préférence Phe ou Leu ; de manière plus particulièrement préférée Phe ; et
   X3 est Phe, Leu, Ile, Tyr, Trp ou Val ; de préférence Phe ou Leu ; de manière plus particulièrement préférée Leu ; et
(ii) le cas échéant, les résidus d'acide aminé, chacun étant indépendamment choisi dans le groupe constitué par Val, Leu, Ile, Phe, Tyr, Trp, Lys, Hyl, Arg et His ; de préférence Val, Leu, Ile, Phe, Tyr et Trp ; de manière plus particulièrement préférée Leu, Ile et Phe.

Quand B et B' comportent plus de 4 résidus non polaires d'acide aminé, A et A comportent de préférence au moins 3 résidus d'acide aminé chargés de manière positive.

Dans C et C', les résidus d'acide aminé peuvent être n'importe quel résidu d'acide aminé à condition que le rapport de résidus d'acide aminé cationique / résidus d'acide aminé hydrophobe demeure dans la gamme indiquée. Avantageusement, ils sont indépendamment choisis parmi les résidus d'acide aminé non-chargés, polaires ou non-polaires, ces derniers étant préférés. Cependant, d'une façon préférée, les positions C et C' sont des positions vides.

Par conséquent, une classe préférée des dimères sont de formule (IV) 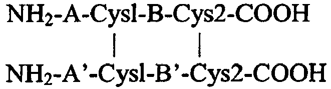 dans laquelle les deux résidus de Cysl sont liés ensemble par un pont disulfure et les deux résidus Cys2 sont liés ensemble par un pont disulfure ;
ou de formule (V) 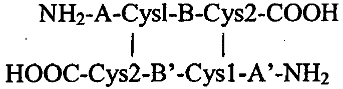 dans laquelle les résidus Cysl sont liés aux résidus Cys2 par des ponts disulfure inter-chaîne peptidique ;
formules (IV) et (V) dans lesquelles A, A', B et B' sont comme décrit ci-dessus ; à condition que le rapport acide aminé cationique / acide aminé hydrophobe dans le dimère de formule (IV) ou (V), soit de 0.4 à 2, avantageusement de 0.5 à 1.2 ou 1.5, de préférence de 0.6 à 1 ; mieux de 0.6 à 0.8 ; par exemple. 0.75.

Dans les formules (II) à (V), A et A' sont de préférence identiques. De même en ce qui concerne B et B' d'une part ; et C et C' d'autre part. Un dimère de formule (II) à (V), dans lesquelles A et A' ; B et B' ; et C et C' sont deux à deux identiques, est désigné sous le nom de dimère homologue.

Pour ces derniers, on cite à titre d'exemple, les dimères parallèle et antiparallèle formés à partir du peptide SAEP2-L2 :

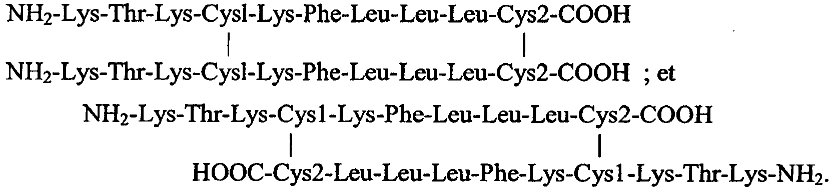

L'endotoxoïde utile aux fins de la présente invention peut être avantageusement caractérisé par un rapport molaire LOS : peptide de 1 : 1.5 à 1 : 0.5, de préférence de 1 : 1.2 à 1 : 0.8, de manière tout particulièrement préférée de 1 : 1.1 à 1 : 0.9, e.g. de 1 : 1.

### LOS détoxifié en liposomes

Lorsque le LOS est formulé en liposomes, il n'apparaît pas forcément nécessaire de le détoxifier au préalable. En effet, le LOS en liposomes - c'est-à-dire associé à la bicouche lipidique formant les liposomes - peut voir sa toxicité décroître de manière très substantielle. L'ampleur de cette décroissance pouvant aller jusqu'à une perte substantielle, est en partie fonction de la nature des composants formant le liposome. Ainsi, lorsque l'on utilise des composants chargés positivement (composants de nature cationique), la perte de toxicité peut être plus importante qu'avec des composants non-chargés (neutre) ou anioniques.

Par « liposomes », on entend une entité synthétique, de préférence une vésicule synthétique, formée d'au moins une membrane (ou matrice) lipidique bi-couche refermée sur un compartiment aqueux. Aux fins de la présente invention, les liposomes peuvent être unilamellaires (une seule membrane bi-couche) ou plurilamellaires (plusieurs membranes disposées en oignon). Les lipides constituant la membrane bi-couche comportent une région non-polaire qui de manière typique est faite de chaîne(s) d'acides gras ou de cholestérol, et d'une région polaire, typiquement faite d'un groupement phosphate et/ou de sels d'ammonium tertiaires ou quaternaires. En fonction de sa composition, la région polaire peut, notamment à pH physiologique (pH ≈ 7), être porteuse d'une charge de surface nette (globale) soit négative (lipide anionique), soit positive (lipide cationique) ou ne pas porter de charge nette (lipide neutre).

Aux fins de détoxification du LOS, les liposomes peuvent être n'importe quel type de liposomes ; en particulier, ils peuvent être constitués de n'importe quel lipide connu pour son utilité dans la fabrication des liposomes. Le ou les lipides entrant dans la composition des liposomes peuvent être des lipides neutres, anioniques ou cationiques ; ces derniers étant préférés. Ces lipides peuvent être d'origine naturelle (produits d'extraction de plante ou d'oeuf, par exemple) ou synthétique ; ces derniers étant préférés. Les liposomes peuvent être aussi constitués d'un mélange de ces lipides ; par exemple d'un lipide cationique ou anionique et d'un lipide neutre, en mélange. Dans ces deux derniers cas, le lipide neutre est souvent désigné sous le terme de co-lipide. Selon un mode de mélange avantageux, le rapport molaire lipide chargé (cationique ou anionique) : lipide neutre est compris entre 10 : 1 et 1 : 10, avantageusement entre 4 : 1 et 1 : 4, de préférence entre 3 : 1 à 1 : 3, bornes incluses.

En ce qui concerne les lipides neutres, on cite à titre d'exemple : (i) le cholestérol ; (ii) les phosphatidylcholines comme par exemple les 1,2-diacyl-sn-glycéro-3-phosphocholines e.g., la 1,2-dioleoyl-*sn*-glycéro-3-phosphocholine (DOPC), ainsi que les 1-acyl-2-acyl-*sn*-glycéro-3-phosphocholines dont les chaînes acyles sont différentes entres elles (chaînes acyles mixtes) ; et (iii) les phosphatidyléthanolamines comme par exemple les 1,2-diacyl-*sn*-glycéro-3-phosphoéthanolamines e.g., la 1,2-dioleoyl-*sn*-glycéro-3-phosphoéthanolamine (DOPE), ainsi que les 1-acyl-2-acyl-*sn*-glycéro-3-phosphoéthanolamine portant des chaînes acyles mixtes.

En ce qui concerne les lipides anioniques, on cite à titre d'exemple (i) l'hémisuccinate de cholestérol (CHEMS) ; (ii) les phosphatidylsérines telles que les 1,2-diacyl-*sn-*glycéro-3-[phospho-L-sérine] e.g., la 1,2-dioleoyl-*sn*-glycéro-3-[phospho-L-sérine] (DOPS), et les 1-acyl-2-acyl-*sn*-glycéro-3-[phospho-L-sérine] portant des chaînes acyles mixtes; (iii) les phosphatidylglycérols tels que les 1,2-diacyl-*sn*-glycéro-3-[phospho-*rac*-(1-glycérol)] *e.g.*, la 1,2-dioleoyl-*sn*-glycéro-3-[phospho-*rac*-(1-glycérol)] (DOPG), et les 1-acyl-2-acyl-*sn*-glycéro-3-[phospho-*rac*-(1-glycérol)] portant des chaînes acyles mixtes ; (iv) les acides phosphatidiques tels que les 1,2-diacyl-*sn*-glycéro-3-phosphate *e.g.,* le 1,2-dioleoyl-*sn*-glycéro-3-phosphate (DOPA), et les 1-acyl-2-acyl-*sn*-glycéro-3-phosphate portant des chaînes acyles mixtes ; et (v) les phosphatidylinositols tels que les 1,2-diacyl-*sn*-glycéro-3-(phospho-inositol) *e.g*., le 1,2-dioleoyl-*sn*-glycéro-3-(phospho-inositol) (DOPI) et les 1-acyl-2-acyl-*sn*-glycéro-3-(phospho-inositol) portant des chaînes acyles mixtes.

En ce qui concerne les lipides cationiques, on cite à titre d'exemple :
(i) les amines lipophiles ou alkylamines comme par exemple le diméthyldioctadécylammonium (DDA), le triméthyldioactadecylammonium (DTA) ou les homologues structuraux du DDA et du DTA [ces alkylamines sont avantageusement utilisées sous forme de sel ; on cite par exemple le bromure de diméthyldioctadécylammonium (DDAB)] ;
(ii) l'octadécenoyloxy(éthyl-2-heptadécenyl-3-hydroxyéthyl) imidazolinium (DOTIM) et ses homologues structuraux ;
(iii) les lipospermines telles que la *N*-palmitoyl-D-erythrospingosyl-1-*O*-carbamoyl spermine (CCS) et la dioctadecylamidoglycylspermine (DOGS, Transfectam) ;
(iv) les lipides incorporant une structure éthylphosphocholine tels les dérivés cationiques des phospholipides, en particulier les dérivés ester phosphoriques de la phosphatidylcholine, par exemple ceux décrits dans la demande de brevet WO 05/049080 et incluant notamment :
   - la 1,2-dimyristoyl-*sn*-glycéro-3-éthylphosphocholine,
   - la 1,2-dipalmitoyl-*sn*-glycéro-3-éthylphosphocholine,
   - la 1,2-palmitoyl-oleoyl-*sn*-glycéro-3-éthylphosphocholine,
   - la 1,2-distearoyl-*sn*-glycéro-3-éthylphosphocholine (DSPC),
   - la 1,2-dioleyl-*sn*-glycéro-3-éthylphosphocholine (DOEPC ou EDOPC ou ethyl-DOPC ou ethyl PC),
   - ainsi que leurs homologues structuraux ;
(v) les lipides incorporant une structure triméthyl ammonium tels que le *N*-(1-[2,3-dioleyloxy]propyl)-*N,N,N*-trimethylammonium (DOTMA) et ses homologues structuraux et ceux incorporant une structure triméthylammonium propane, tels que le 1,2-dioleyl-3-triméthylammonium propane (DOTAP) et ses homologues structuraux ; ainsi que les lipides incorporant une structure diméthylammonium tels que le 1,2-dioleyl-3-diméthylammonium propane (DODAP) et ses homologues structuraux ; et (vi) les dérivés cationiques du cholestérol tels que le 3β-[N-(N',N'-dimethylaminoethane)-carbamoyl] cholestérol (DC-Chol) ou d'autres dérivés cationiques du cholestérol comme ceux décrits dans le brevet US 5 283 185 et notamment l'iodure de cholestéryl-3β-carboxamidoéthylènetriméthylammonium, la cholestéryl-3β-carboxyamidoéthylènamine, l'iodure de cholestéryl-3β-oxysuccinamido-éthylènetriméthylammonium et le 3β-[N-(polyéthylènimine)-carbamoyl]-cholestérol.

Par « homologues structuraux », on signifie les lipides qui possèdent la structure caractéristique du lipide de référence tout en s'en différentiant par des modifications secondaires, notamment au niveau de la région non-polaire, en particulier du nombre d'atomes de carbone et des doubles liaisons des chaînes d'acides gras.

Ces acides gras, que l'ont trouve aussi dans les phospholipides neutres et anioniques, sont par exemple l'acide dodécanoique ou laurique (C12 :0), l'acide tétradécanoique ou myristique (C14:0), l'acide hexadécanoique ou palmitique (C16:0), l'acide cis-9-hexadecanoique ou palmitoleique (C16:1), l'acide octadécanoique ou stéarique (C18:0), l'acide cis-9-octadécanoique ou oleique (C18:1), l'acide cis,cis-9,12-octadécadiénoiquec ou linoléique (C18:2), l'acide cis-cis-6,9-octadécadiénoique (C18:2), l'acide all cis-9,12,15-octadécatriénoique ou α-linolénique (C18 :3), l'acide all cis-6,9,12-octadécatriénoique ou γ-linolénique (C18:3), l'acide eicosanoique ou arachidique (C20 :0), l'acide cis-9-eicosénoique ou gadoleique (C20:1), l'acide all cis-8,11,14-eicosatrienoique (C20:3), l'acide all cis-5,8,11,14-eicosatetraenoique ou arachidonique (C20 :4), l'acide all cis-5,8,11,14,17-eicosapentaneoique (C20 :5), l'acide docosanoique ou behenique (C22 :0), l'acide all cis-7,10,13,16,19-docosapentaenoique (C22 :5), l'acide all cis-4,7,10,13,16,19-docosahexaenoique (C22 :6) et l'acide tétracosanoique ou lignocérique (C24 :0).

Selon un mode particulier, on utilise un mélange de lipide cationique et de lipide neutre. A titre d'exemple, on cite :
- un mélange de DC-chol et de DOPE, notamment dans un rapport molaire DC-chol : DOPE allant de 10 : 1 à 1 : 10, avantageusement de 4 : 1 à 1 : 4, de préférence d'environ 3 : 1 à 1 : 3;
- un mélange d'ethyl-DOPC et de cholestérol, notamment dans un rapport molaire ethyl-DOPC : cholestérol allant de 10 : 1 à 1 : 10, avantageusement de 4 : 1 à 1 : 4, de préférence d'environ 3 : 1 à 1 : 3; et
- un mélange d'ethyl-DOPC et de DOPE, notamment dans un rapport molaire ethyl-DOPC : DOPE allant de 10: 1 à 1 : 10, avantageusement de 4: 1 à 1 : 4 de préférence d'environ de 3 : 1 à 1 : 3.

Selon un mode de préparation avantageux, on réalise dans un premier temps, un film lipidique sec avec tous les composés entrant dans la composition des liposomes. Puis on reconstitue le film lipidique en milieu aqueux, en présence de LOS, par exemple dans un rapport molaire lipides : LOS de 100 à 500, de manière avantageuse de 100 à 400 ; de préférence de 200 à 300 ; de manière tout particulièrement préférée, de 250 environ. D'une manière générale, on estime que ce même rapport molaire ne devrait pas varier de manière substantielle en final du procédé de préparation des liposomes LOS.

De manière générale, l'étape de reconstitution en milieu aqueux conduit à la formation spontanée de vésicules multi-lamellaires dont la taille est ensuite homogénéisée par diminution progressive du nombre de lamelles par extrusion, par exemple à l'aide d'un extrudeur par passages de la suspension lipidique sous pression d'azote, à travers des membranes en polycarbonates ayant des diamètres de pores de plus en plus réduits (0.8, 0.4, 0.2 µm). On peut aussi remplacer le procédé d'extrusion par un autre procédé mettant en oeuvre un détergent (tensio-actif) qui disperse les lipides. Ce détergent est ensuite éliminé par dialyse ou par adsorption sur des microbilles de polystyrène poreuses particulièrement affines de détergent (BioBeads). Quand le tensio-actif se retire de la dispersion lipidique, les lipides se réorganisent en double couche.

A l'issue de l'incorporation du LOS en liposomes, on peut communément obtenir un mélange constitué de liposomes *adhoc* et de LOS forme libre. Avantageusement, les liposomes sont alors purifiés afin de se débarrasser du LPS non-détoxifié sous forme libre.

### Conjugaison du LOS

Dans un vaccin selon l'invention, le LOS est avantageusement sous forme de conjugué LOS-polypeptide porteur, notamment quand il n'est pas sous forme d'OMVs ni de liposomes.

Le polypeptide porteur peut être n'importe quel polypeptide, oligopeptide ou protéine porteuse en usage dans le domaine des vaccins conjugués ; et notamment l'anatoxine *pertussis,* diphtérique ou tétanique, le mutant de la toxine diphtérique dénommé CRM197, une OMP bactérienne, un complexe bactérien de protéine [par exemple l'OMPC (outer-membrane protein C) de *N. meningitidis],* l'exotoxine A de *Pseudomonas,* la lipoprotéine D *d'Haemophilus influenzae,* la pneumolysine de *Streptococcus pneumoniae,* l'hémagglutinine filamenteuse de *Bordetella pertussis* et la sous-unité B du récepteur de la transferrine humaine de *N. meningitidis.*

De nombreuses méthodes de conjugaison existent dans le domaine technique. Certaines sont répertoriées par exemple dans les demandes de brevet EP 941 738 et WO 98/31393.

D'une manière générale, les groupements réactifs du LOS mis en jeu lors de la conjugaison sont ceux du core interne (inner core) ou du lipide A. Il peut s'agir entre autre de la fonction acide du KDO ou bien d'un aldéhyde généré suite à un traitement approprié sur le disaccharide du lipide A. Par exemple, un traitement à la phosphatase génère un aldéhyde sur la structure de la deuxième glucosamine du lipide A de *N. meningitidis* (Brade H. (2002) J. Endotoxin Res. 8 (4) : 295 Mieszala et al, (2003) Carbohydrate Res. 338 : 167 et Cox et al, (2005) Vaccine 23 (5) : 5054).

De manière avantageuse, le procédé de conjugaison fait usage (i) d'un agent de liaison bifonctionnel (linker) ou (ii) d'un espaceur et d'un linker.

Par exemple, dans le premier cas, on active le LOS avec un agent de couplage bifonctionnel (linker) de formule R1 - A - R2 de telle sorte que le radical R2 réagisse avec un groupement réactif du KDO ou du lipide A afin d'obtenir un LOS activé ; puis on conjugue le LOS activé avec le polypeptide de telle sorte que le substituant R1 réagisse avec un groupement fonctionnel porté par le polypeptide afin d'obtenir un conjugué.

Par exemple, dans le deuxième cas, on dérive le LOS avec un espaceur de formule R3 - B - R4 de telle sorte que le radical R3 réagisse avec un groupement réactif du KDO ou du lipide A afin d'obtenir un LOS dérivé ; puis on active le LOS dérivé avec un agent de couplage bifonctionnel (linker) de formule R1 - A - R2 de telle sorte que le radical R2 réagisse avec le radical R4 afin d'obtenir un LOS dérivé et activé ; enfin on conjugue le LOS dérivé et activé avec le polypeptide de telle sorte que le radical R1 réagisse avec un groupement fonctionnel porté par le polypeptide afin d'obtenir un conjugué.

Dans le deuxième cas on peut aussi procéder de la manière suivante : On dérive le polypeptide avec un espaceur de formule R3 - B - R4 de telle sorte que le radical R4 réagisse avec un groupement fonctionnel porté par le polypeptide ; on active le LOS avec un agent bifonctionnel (linker) de formule R1 - A - R2 de telle sorte que le radical R2 réagissent avec un groupement réactif du KDO ou du lipide A afin d'obtenir un LOS activé ; puis, on conjugué le LOS activé avec le polypeptide dérivé de telle sorte que le radical R1 du LOS activé réagisse avec le radical R3 du polypeptide dérivé afin d'obtenir un conjugué.

Dans la formule de l'espaceur, B peut être une chaîne carbone, de préférence carbonyl, alkyl ou alkylène, par exemple en C1 à C12. R3 et R4 peuvent être de manière indépendante un groupe thiol ou amine ou un résidu le portant, par exemple un groupe hydrazide i.e., NH₂-NH-CO-. Des composés pouvant être utilisés comme espaceur sont par exemple de formule NH₂ - B - NH2, ou de préférence NH₂ - B - SH et NH₂ - B - S - S - B' - NH₂. A titre d'exemple particulier on cite : la cystéamine, la cystéine les diamines e.g., le diaminohexane, l'acide adipique dihydrazide (ADH) l'urée et la cystamine.

Dans la formule du linker, A peut être une chaîne aromatique ou de préférence aliphatique substituée ou non, qui de manière avantageuse, comprend de 1 à 12 atomes de carbone ; de préférence 3 à 8 atomes de carbone. Par exemple A peut être un C2 à C8 alkylène, un phénylène, un C7 à C12 aralkylène, un C2 à C8 alkyle, un phényle, un C7 à C12 aralkyle, C6 alkanoyloxy ou un benzylcarbonyloxy, substitué ou non.

Le radical R2 est le groupement fonctionnel du linker qui crée le lien au LOS ou au LOS dérivé. Ainsi, R2 est un groupement fonctionnel qui peut réagir avec un groupe carboxyle, hydroxyle, aldéhyde ou amine. Si le linker doit réagir avec un groupe hydroxyle carboxyle ou aldéhyde, R2 est de préférence un groupe amine ou un résidu le portant, par exemple un groupe hydrazide *i.e*., NH₂-NH-CO-. Si le linker doit réagir avec un groupe amine, R2 est de préférence un groupe carboxyl, succinimidyl (*e.g*., N-hydroxy succinimidyl) ou sulfosuccinimidyl (*e.g*., N-hydroxy sulfosuccinimidyl).

Ainsi, des composés pouvant être utilisés comme linker peuvent être choisis parmi l'acide adipique dihydrazide (ADH); le sulfosuccinimidyl-6-(3-[2-pyridyldithio] propionamido)-hexanoate (Sulfo-LC-SPDP) ; le succinimidyl-6-(3-[2-pyridyldithio] propionamido)-hexanoate (LC-SPDP) ; le N-succinimidyl-S-acetyl thioacetate (SATA) ; le N-Succinimidyl-3-(2-pyridyl dithio) propionate (SPDP), succinimidyl acetyl thiopiopionate (SATP) ; le succinimidyl-4-(N-maleimido methyl) cyclohexane-1-carboxylate (SMCC) ; le maleimido benzoyl-N-hydroxy succinimide ester (MBS) ; le N-succinimidyl (4-iodoacetyl) aminobenzoate (SIAB); le succinimidyl 4-(p-maleimidophenyl) butyrate (SMPB) ; le bromoacétique acide -N-hydroxy succinimide (BANS) ester ; le dithiobis (succinimidyl propionate) (DTSSP); le H-(γ-maléimido butyryloxy) succinimide ester (GMBS) ; le succinimidyl-4-(N-maléimidométhyl) cyclohexane-1-carboxylate; le N-succinimidyl-4-(4-maléimidophényl) butyrate; le N-[ß-maleimidocaproic acid] hydrazide (BMCH) ; le N-succinimidyl-4-maléimido-butyrate ; et le N-succinimidyl-3-maléiimido-benzoate.

A titre d'exemple, on propose d'utiliser la fonction acide du KDO pour dériver le LOS avec de l'ADH en présence d'un carbodiimide [*e.g*., 3-diméthylaminopropyl)-3-ethyle carbodiimide hydrochloride (EDAC)]. Puis on fait réagir la fonction amine ainsi introduite avec les fonctions carboxyles du polypeptide, en présence d'EDAC, après avoir protégé les fonctions amines de ce dernier (Wu et al (2005) Vaccine 23 : 5177) ou les avoir transformées en fonctions acide (Succinylation de la protéine ; Pavliakova et al, Infect. Immun. (1999) 67 (10): 5526).

De manière alternative, on propose d'utiliser la fonction acide du KDO pour dériver le LOS avec de la cystéamine ou de la cystéine en présence d'EDAC. Puis on fait réagir la fonction thiol ainsi introduite avec la fonction maléimide d'un linker homobifonctionnel, tel que le bis maleimido hexane ; ou hétérobifonctionnel, tel que le GMBS. Dans le premier cas, on fait alors réagir la fonction maléimide ainsi introduite avec les fonctions thiols du polypeptide. Dans le deuxième cas, on fait réagir la fonction succinimidyle du LOS dérivé et activé avec les fonctions amines du polypeptide.

En fonction du mode de conjugaison retenu, le LOS et le polypeptide peuvent être conjugués l'un à l'autre dans un rapport molaire LOS : polypeptide de 10⁻¹ à 10², de manière avantageuse de 1 à 10², de préférence de 1 à 50 ; de manière tout particulièrement préférée de 20 environ.

Comme annoncé précédemment, l'invention a pour objet une composition pharmaceutique à l'encontre des infections à *N*. *meningitidis,* notamment de sérogroupe B, qui comprend :
(i) Un LOS de *N. meningitidis,* constitué notamment d'un lipide A non-détoxifié, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ou un LOS obtenu selon le procédé de préparation (ii) ; et, de manière optionnelle,
(ii) Un LOS de *N. meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ou un LOS obtenu selon le procédé de préparation (i) ; et, de manière optionnelle,
(iii) Un LOS de *N. meningitidis* constitué notamment d'un lipide A, d'un core interne, d'une chaîne α de type L6 ou L8, dans lequel le résidu heptose II du core interne porte en position O-6 ou O-7 un substituant phosphoéthanolamine (PEA) et ne porte pas de substituant PEA en position O-3.

L'invention a aussi pour objet une composition pharmaceutique à l'encontre des infections à *N*. *meningitidis,* notamment de sérogroupe B, qui comprend un LOS de *N. meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ; ou obtenu selon le procédé de préparation (i).

Comme précédemment indiqué, cette dernière composition peut comprendre en outre un LOS obtenu selon le procédé de préparation (ii) ou un LOS de *N. meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; ainsi qu'un LOS de *N. meningitidis* constitué notamment d'un lipide A, d'un core interne, d'une chaîne α de type L6, dans lequel le résidu heptose II du core interne porte en position O-6 ou O-7 un substituant phosphoéthanolamine (PEA) et ne porte pas de substituant PEA en position O-3.

L'invention a aussi pour objet une composition pharmaceutique à l'encontre des infections à *N. meningitidis,* notamment de sérogroupe B, qui comprend :
(i) Un LOS de *N. meningitidis,* de préférence de sérogroupe A, constitué notamment d'un lipide A, d'un core interne, d'une chaîne α de type L8, dans lequel le résidu heptose II du core interne porte en position O-3 un substituant phosphoéthanolamine (PEA) et ne porte pas de substituant PEA en positions O-6 et O-7 ; et
(ii) Un LOS de *N. meningitidis,* constitué notamment d'un lipide A non-détoxifié, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ou un LOS obtenu selon le procédé de préparation (ii) ; ou un LOS de *N. meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ; ou obtenu selon le procédé de préparation (i).

Dans cette dernière composition, le LOS du point (i) peut être de manière avantageuse le LOS d'une souche d'immunotype L8.

L'invention a aussi pour objet une composition pharmaceutique à l'encontre des infections à *N*. *meningitidis,* notamment de sérogroupe B, qui comprend :
(i) Un LOS de *N. meningitidis,* de préférence de sérogroupe A, constitué notamment d'un lipide A, d'un core interne, d'une chaîne α de type L8, dans lequel le résidu heptose II du core interne porte en position O-3 et en position O-6 ou O-7 un substituant phosphoéthanolamine (PEA) ; et
(ii) Un LOS de *N. meningitidis,* constitué notamment d'un lipide A non-détoxifié, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ou un LOS obtenu selon le procédé de préparation (ii) ; ou un LOS de *N. meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ; ou obtenu selon le procédé de préparation (i).

Dans cette dernière composition, le LOS du point (i) peut être de manière avantageuse le LOS de la souche C708 *lpt3FL lpt6TR lgtA::erm* ou le LOS de la souche A1 (Zhu, Klutch & Tsai, FEMS Microbiology Letters (2001) 203 : 173 ainsi que Gu, Tsai & Karpas, J. Clin. Microbiol. (Aug 1992) 30 (8) : 2047).

Ainsi, une composition vaccinale *i.a.*, bivalente, selon l'invention peut se décliner selon des modes variés. On cite les 6 exemples suivants :
1) Une composition vaccinale selon l'invention, qui comprend :
   (i) un LOS obtenu selon le procédé de préparation (i) ou un LOS de *N*. *meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ; et
   (ii) un LOS obtenu selon le procédé de préparation (ii) ou un LOS de *N*. *meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7.
2) Une composition vaccinale selon l'invention, qui comprend :
   (i) un LOS obtenu selon le procédé de préparation (i) ou un LOS de *N*. *meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; et
   (ii) un LOS de *N. meningitidis* constitué notamment d'un lipide A, d'un core interne, d'une chaîne α de type L6, dans lequel le résidu heptose II du core interne porte en position O-6 ou O-7 un substituant phosphoéthanolamine (PEA) et ne porte pas de substituant PEA en position O-3.
3) Une composition vaccinale selon l'invention, qui comprend :
   (i) un LOS obtenu selon le procédé de préparation (i) ou un LOS de *N*. *meningitidis* constitué notamment d'un lipide A, d'un core interne, d'une chaîne α de type L6, dans lequel le résidu heptose II du core interne porte en position O-3 et ne porte pas de substituant phosphoéthanolamine (PEA) en positions O-6 et O-7 ; et
   (ii) un LOS de *N*. *meningitidis* constitué notamment d'un lipide A, d'un core interne, d'une *chaîne* α de type L8, dans lequel le résidu heptose II du core interne porte en position O-3 un substituant phosphoéthanolamine (PEA) et ne porte pas de substituant PEA en positions O-6 et O-7.
(4) Une composition vaccinale selon l'invention, qui comprend :
   (i) un LOS obtenu selon le procédé de préparation (i) ou un LOS de *N. meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA) ; et
   (ii) un LOS de *N. meningitidis* constitué notamment d'un lipide A, d'un core interne, d'une *chaîne* α de type L8, dans lequel le résidu heptose II du core interne porte en position O-3 un substituant phosphoéthanolamine (PEA) et ne porte pas de substituant PEA en positions O-6 et O-7.
5) Une composition vaccinale selon l'invention, qui comprend :
   (i) un LOS obtenu selon le procédé de préparation (iii) ou un LOS de *N*. *meningitidis* constitué notamment d'un lipide A, d'un core interne, d'une chaîne α de type L8, dans lequel le résidu heptose II du core interne porte en position O-3 et en position O-6 ou O-7 un substituant phosphoéthanolamine (PEA) ; et
   (ii) un LOS obtenu selon le procédé de préparation (ii) ou un LOS de *N*. *meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7.
6) Une composition vaccinale selon l'invention, qui comprend :
   (i) un LOS obtenu selon le procédé de préparation (iii) ou un LOS de *N*. *meningitidis* constitué notamment d'un lipide A, d'un core interne, d'une chaîne α de type L8, dans lequel le résidu heptose II du core interne porte en position O-3 et en position O-6 ou O-7 un substituant phosphoéthanolamine (PEA) ; et
   (ii) un LOS obtenu selon le procédé de préparation (ii) ou un LOS de *N*. *meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA).

Une composition vaccinale / pharmaceutique selon l'invention est notamment utile pour traiter ou prévenir une infection à *N*. *meningitidis,* telles que les méningites à *N*. *meningitidis,* les méningococcémies et les complications qui peuvent en dériver telles que le *purpura fulminans* et le choc septique ; ainsi que les arthrites et péricardites à *N*. *meningitidis.*

Elle peut être fabriquée de manière conventionnelle. En particulier, on associe une quantité efficace d'un point de vue thérapeutique ou prophylactique du constituant essentiel du vaccin qui est le LOS, à un support ou à un diluant acceptable d'un point de vue pharmaceutique. Avantageusement, elle peut en outre comprendre un adjuvant acceptable d'un point de vue pharmaceutique.

Pour usage dans une composition selon l'invention, le (les) LOS est (sont) avantageusement formulé(s) en liposomes.

De manière additionnelle, une composition selon l'invention peut comprendre un ou plusieurs antigène(s) vaccinal(aux) additionnel(s) de *N. meningitidis ;* par exemple un ou des polypeptides de *N. meningitidis.* Sous une forme particulièrement préférée, une composition selon l'invention peut comprendre (i) la sous-unité B (TbpB) du récepteur de la transferrine humaine qui est une lipoprotéine de la membrane externe d'un certain nombre de bactéries à Gram-négatif non-entériques telles que les *Neisseriae, e.g. N*. *meningitidis* ; ou (ii) un fragment N-terminal lipidé de cette dernière. Dans ce cas là, la TbpB lipidée ou un fragment lipidé de cette dernière peut à la fois remplir son office d'antigène vaccinal ainsi que celui d'adjuvant du LOS.

Chez *N. meningitidis* et pour ce qui concerne la TbpB, les souches se répartissent en deux isotypes : les isotypes I et II qui diffèrent selon la longueur de la chaîne d'acides aminés (EP 560 969 et EP 586 266). Pour l'isotype I, la souche de référence est la souche B16B6. Pour l'isotype II, la souche de référence est la souche M982.

Selon un mode avantageux, une composition selon l'invention comprend de manière additionnelle la TbpB lipidée d'une souche de *N. meningitidis* d'isotype I ou d'isotype II ou la TbpB lipidée de chacune des souches d'isotype I et II. A cette fin, la TbpB lipidée d'une souche de *N. meningitidis* d'isotype I peut être celle de la souche B16B6 ; et la TbpB lipidée d'une souche de *N. meningitidis* d'isotype II peut être celle de la souche M982.

Ainsi, à titre d'exemple, on cite :
1) Une composition vaccinale selon l'invention, qui comprend :
   (i) un LOS obtenu selon le procédé de préparation (ii) ou un LOS de *N*. *meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; et
   (ii) la TbpB lipidée d'une souche de *N*. *meningitidis* d'isotype II ou un fragment N-terminal lipidé de cette dernière.
2) Une composition vaccinale selon l'invention, qui comprend :
   (i) un LOS obtenu selon le procédé de préparation (ii) ou un LOS de *N*. *meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; et
   (ii) la TbpB lipidée d'une souche de *N*. *meningitidis* d'isotype II ou un fragment N-terminal lipidé de cette dernière ; et
   (iii) la TbpB lipidée d'une souche de *N. meningitidis* d'isotype I ou un fragment N-terminal lipidé de cette dernière.
3) Une composition vaccinale selon l'invention, qui comprend :
   (i) un LOS obtenu selon le procédé de préparation (ii) ou un LOS de *N*. *meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; et
   (ii) un LOS de *N*. *meningitidis* constitué notamment d'un lipide A, d'un core interne, d'une chaîne α de type L8, dans lequel le résidu heptose II du core interne porte en position O-3 un substituant phosphoéthanolamine (PEA) et ne porte pas de substituant PEA en positions O-6 et O-7 ; ou un LOS obtenu selon le procédé de préparation (iii) ou un LOS de *N*. *meningitidis* constitué notamment d'un lipide, A, d'un core interne, d'une chaîne α de type L8, dans lequel le résidu heptose II du core interne porte en position O-3 et en position O-6 ou O-7 un substituant phosphoéthanolamine (PEA) ; et
   (iii) la TbpB lipidée d'une souche de *N. meningitidis* d'isotype II ou un fragment N-terminal lipidé de cette dernière ; et de manière optionnelle,
   (iv) la TbpB lipidée d'une souche de *N. meningitidis* d'isotype I ou un fragment N-terminal lipidé de cette dernière.

Lorsqu'une composition vaccinale selon l'invention comprend une ou plusieurs TbpB, cette (ces) dernière (s) peut (peuvent) être formulées avec le LOS en liposomes ou bien être en simple mélange avec des liposomes LOS (LOS formulé en liposomes).

Les quantités de LOS par dose de vaccin qui sont suffisantes pour atteindre les fins pré-citées, efficaces d'un point de vue immunogène, prophylactique ou thérapeutique, dépendent de certains paramètres incluant l'individu traité (adulte, adolescent, enfant ou nourrisson), la voie d'administration et de sa fréquence.

La quantité de LOS par dose, suffisante pour atteindre les fins pré-citées est notamment comprise entre 5 et 500 µg, avantageusement entre 10 et 200 µg, de préférence entre 20 et 100 µg, de manière tout à fait préférée entre 20 et 80 µg ou entre 20 et 60 µg, bornes incluses.

Le terme « dose » employé ci-dessus doit être entendu comme désignant un volume de vaccin administré à un individu en une seule fois - c'est-à-dire à un temps T. Des doses conventionnelles sont de l'ordre du millilitre, par exemple 0.5, 1 ou 1.5 ml ; le choix définitif dépendant de certains paramètres et notamment de l'âge et du statut du receveur. Un individu peut recevoir une dose répartie en plusieurs sites d'injection le même jour. La dose peut être unique ou si nécessaire, l'individu peut aussi recevoir plusieurs doses à un certain délai d'intervalle - délai d'intervalle pouvant être déterminé par l'homme de l'art.

Elle peut être administrée par n'importe quelle voie conventionnelle en usage dans le domaine de l'art, *e.g.* dans le domaine des vaccins, notamment par voie entérale ou parentérale. L'administration peut avoir lieu en dose unique ou répétée avec un certain délai d'intervalle. La voie d'administration varie en fonction de divers paramètres par exemple de l'individu traité (condition, âge, etc.).

Enfin l'invention a également pour objet :
- Une méthode pour induire chez un mammifère, par exemple un humain, une réponse immune à l'encontre de *N. meningitidis,* selon laquelle on administre au mammifère une quantité efficace d'un point de vue immunogénique d'une composition selon l'invention pour induire une réponse immune, en particulier une réponse immune protectrice à l'encontre de *N. meningitidis* ; et
- Une méthode de prévention et/ou de traitement d'une infection induite par *N*. *meningitidis,* selon laquelle on administre une quantité efficace d'un point de vue prophylactique ou thérapeutique d'une composition selon l'invention à un individu ayant besoin d'un tel traitement.

### DONNEES EXPERIMENTALES

### A Données expérimentales relatives aux souches dérivées de N. meningitidis C708

### 1. Matériel & Méthodes

### 1.1 Transformation de la souche C708

La souche C708 est cultivée en milieu agar BHI (Brain Heart Infusion) à 37°C sous une atmosphère à 10 % CO₂. La nappe bactérienne est récoltée en milieu liquide BHI complémenté en MgCl₂ 5 mM pour obtenir une suspension bactérienne à 10⁹ cfu/mL (cfu : colony-forming-unit ou colonie formant unité).

A 900 µL du milieu liquide BHI + MgCl₂ 5 mM, on ajoute 10 µg d'ADN nécessaire à la transformation (plasmide linéarisé) ; puis 100 µl de la suspension bactérienne (10⁸ germes). Le milieu de transformation est incubé 30 min à 37°C, 10 % CO₂.

Cinq cent µL de cette préparation (soit environ 5.10⁷ cfu) servent à ensemencer 4,5 mL de BHI + MgCl₂ 5 mM. On laisse les bactéries se régénérer 2 hrs à 37°C, 10 % CO₂. Puis, à partir de cette suspension, on réalise des dilutions en BHI + MgCl₂ 5 mM. 300 µL d'une dilution contenant environ 30 000 cfu sont étalés sur boîte BHI agar 140 mm. Les boîtes sont placées à 37°C, 10 % CO₂ pendant au moins 20 hrs.

### 1.2. Buvardage des colonies de transformants

Les colonies sont transférées sur membrane Hybond-XL 137 mm (GE Healthcare; #RPN 137S). Les germes déposés sur membrane sont lysés en tampon de dénaturation (NaOH 0.5 M, NaCl 1,5 M). Les membranes sont lavées en tampon de neutralisation (Tris 0,5 M, NaCl 1,5 M, pH 7.5) ; transférées en milieu SSC 2X ; puis séchées. L'ADN est fixé par incubation 2 hrs à 80°C.

### 1.3. Détection des transformants par hybridation avec une sonde marquée au ³³P dCTP

Le marquage de la sonde est obtenu par amplification PCR en utilisant le kit Ready-to-Go PCR Beads (GE Healthcare) ; puis la sonde marquée est purifiée sur colonne ProbeQuant G50 Microcolumn (GE Healthcare).

Les membranes à hybrider sont placées par trois dans 50 ml de tampon Rapid Hyb (GE Healthcare), 15 min à 65°C, sous agitation lente, pour pré-hybridation. La sonde marquée et dénaturée au préalable 2 min à 95°C, est ajoutée aux membranes. En final, la concentration de la sonde est de 5 ng/ml. On laisse l'hybridation se poursuivre 2 hrs à 65°C, sous agitation lente.

Les membranes sont ensuite soumises à des lavages successifs en procédant comme suit :
- En tampon de faible stringence (2 X SSC, 0.1 % SDS (poids / vol) 15 min à température ambiante sous agitation lente ;
- En tampon de moyenne stringence (1 X SSC, 0.1 % SDS (poids / vol) 20 min à 65°C sous agitation lente ; et
- En tampon de faible stringence (0.1 X SSC, 0.1 % SDS (poids / vol) 45 min à 65°C sous agitation lente.

Une fois séchées, les membranes sont révélées par autoradiographie (film Biomax MR).

### 1.4. Détection des transformants par hybridation avec un oligonucléotide marqué au [γ³²P] ATP

On réalise le marquage de l'extrémité 5' de l'oligonucléotide dans le milieu réactionnel suivant (les quantités indiquées sont celles correspondant à l'hybridation d'une quantité d'oligonucléotide nécessaire à l'hybridation de 3 membranes dans une boîte) :

| | | |
|---|---|---|
| - Oligonucléotide 5'-OH libre | 3 µl maxi | soit 10 pmoles |
| - Tampon de phosphorylation 10 X | 1 µl | soit 1 X |
| - [γ³²P] ATP 10 mCi/ml | 5 µl | soit 50 µCi |
| - T4 kinase (10 U/µl) | 1 µl | soit 10 U |
| - H₂O | qsp 10 µl | |

Le milieu réactionnel est incubé 30 min à 37°C. Puis la T4 kinase est inactivée par chauffage 10 min à 70°C.

Les membranes à hybrider sont placées par trois dans 60 mL de tampon Rapid Hyb (GE Healthcare), 15 min à 48°C, sous agitation lente, pour pré-hybridation. Le tampon de pré-hybridation est éliminé, remplacé par 50 mL du tampon d'hybridation suivant : 5 X SSC, 5 X Denhardt's solution, 0.5 % de SDS (poids/vol.) et 100 µg/ml d'ADN de sperme de saumon à 10 mg/ml soniqué et dénaturé 5 min à 100°C.

L'oligonucléotide marqué (10 µl) est ajouté aux membranes. On laisse l'hybridation se poursuivre une nuit à une température inférieure de 5°C au Tm de l'oligonucléotide, sous agitation lente.

Les membranes sont ensuite soumises à des lavages successifs en procédant dans l'ordre comme suit :
- En tampon de faible stringence (2 X SSC, 0.1 % SDS (poids / vol) 5 min à Tm de l'oligonucléotide - 5°C, sous agitation lente ;
- En tampon de moyenne stringence (1 X SSC, 0.1 % SDS (poids / vol) 15 min à Tm de l'oligonucléotide - 5°C, sous agitation lente ; et
- En tampon de faible stringence (0.1 X SSC, 0.1 % SDS (poids / vol) 10 min à Tm de l'oligonucléotide - 5°C, sous agitation lente.

Une fois séchées, les membranes sont révélées par autoradiographie (film Biomax MR).

### 2. Construction d'une souche de N. meningitidis exprimant un LOS dont la chaîne α est celle d'un LOS d'immunotype L6 et comportant dans chacune des positions 03 et 06 du résidu heptose II (hep II) du core interne, un substituant phosphoéthanolamine (PEA)

On utilise pour souche de départ, la souche de *N. meningitidis* C708 de sérogroupe A et d'immunotype L6 possédant entre autres les caractéristiques suivantes :
- Un gène *lgt*A actif (gène allumé « ON ») ;
- Un gène *lgt*B *-* (gène non fonctionnel) ;
- Un gène *lgt*G éteint (« OFF ») ;
- Un gène *lpt*3 tronqué ;
- Un gène *lpt*6 actif ; et
- Un gène *lot*3 actif.

La souche C708 a été déposée le 11 mars 2008, auprès de la Collection Nationale de Culture de Microorganisme, 25 rue du Dr Roux 75015 Paris, selon les termes du traité de Budapest. Cette souche porte le numéro d'ordre CNCM I-3942.

La souche C708 comporte un gène *lpt3* tronqué. Pour la modifier de telle sorte que le LOS soit porteur d'un substituant PEA en position 03, on choisit de remplacer par recombinaison homologue, le gène *lpt*3 tronqué par le gène *lpt*3 complet (full-length) de la souche de *N. meningitidis* FAM18 sérogroupeC (souche mise à disposition dans les laboratoires de recherche, à l'échelle mondiale). La souche qui en résulte sera dénommée plus commodément C708 *lpt*3 FL.

### 2.1. Amplification par PCR (polymerase chain reaction) du gène lpt3 complet (FL = full-length) de la souche de N. meningitidis FAM18

Cent ng d'ADN génomique de la souche FAM18 ont été utilisés pour amplification avec la Platinum® *Taq* DNA polymerase High Fidelity (Invitrogen, #11304-011).

Le couple d'amorces est le suivant (couple n°1) :
CG GAATTC GCC GTC TCA A ATG AAA AAA TCC CTT TTC GTT CTC (Tm = 55,9°C) ; et
AA CTGCAG TCA TTG CGG ATA AAC ATA TTC CG (Tm = 57,1°C). (sont respectivement soulignés les sites *Eco*RI et *Pst*I).

Pour amplification le mélange suivant a été réalisé :

| Composant | Volume | Concentration finale |
|---|---|---|
| Tampon 10X High Fidelity PCR | 5 µl | 1X |
| Mélange 10 mM dNTP | 1 µl | 0.2 mM de chaque |
| MgSO₄ 50 mM | 2 µl | 2 mM |
| Mélange d'amorces (10 µM chacune) | 1 µl | 0.2 µM de chaque |
| ADN génomique | x µl | 100 ng |
| Platinum® *Taq* High Fidelity | 0.2 µl | 1.0 unit |
| Eau dépourvue de nucléase | pour 50 µl final | Ne s'applique pas |

Le programme du thermocycleur est le suivant :

| | | |
|---|---|---|
| Dénaturation initiale : | | 94°C pendant 30 secondes |
| 30 cycles de : | Dénaturation : | 94°C pendant 30 secondes |
| | Hybridation : | 55°C pendant 30 secondes |
| | Extension : | 68°C pendant 1 minute / kb de produit PCR. |

Après la réaction, 1/10 du produit PCR a été déposé sur gel d'agarose pour vérification.

### 2.2. Construction d'un vecteur de transformation

Le produit PCR d'une part et le plasmide pUC19 d'autre part ont été soumis à une double digestion par *Eco*RI et *Pst*I pendant 2 hrs à 37°C. On a utilisé 10 unités de chaque enzyme par µg d'ADN dans le tampon REact2 (Invitrogen).

Le fragment PCR a été ensuite inséré dans le vecteur pUC19 linéarisé. Les ligations ont été réalisées sous un volume final de 20 µl avec 50 ng de vecteur, 0,5 U de T4 DNA ligase (Invitrogen) et 1 µl d'ATP 10 mM (Invitrogen) pendant 16 heures à 16°C. La ligase a ensuite été inactivée par chauffage 10 min à 65°C.

Le vecteur ainsi obtenu a été transféré par la technique d'électroporation dans une souche d'*E*. *coli XL1* blue MRF résistante à la kanamycine et rendue électro compétentes. Les paramètres suivis pour l'électroporation sont les suivants : Capacitance : 500 µFD ; Résistance : 200 ohms ; Voltage : 1700 volts.

La sélection des clones transformés a été faite par étalement sur boîtes LB ampicilline 100 µg/ml. L'authentification de 10 des 50 clones positifs a été réalisée par amplification PCR. 100 % des clones présentaient le profil attendu. Finalement, le gène *lpt*3 dans un plasmide d'un de ces clones (plasmide pM1222) a été vérifié par séquençage.

### 2.3. Transformation de la souche C708 et détection de l'évènement de recombinaison homologue

### 2.3.1. Transformation

40 µg de pM1222 ont été digérés par 400 U d'*Eco*RI et 10 µg ont été utilisés pour transformer la souche C708 selon la méthode décrite dans la section A.1.1.

Après transformation, les bactéries ont été étalées sur 17 boîtes de Pétri de 140 mm à une concentration théorique de 30 000 cfu par boîte ; soit 510 000 cfu (colonies-forming-units), puis ont été placées une nuit à 37°C. Les boîtes ont ensuite été placées 30 min à +4°C.

Après 24 heures à 37°C, la numération des boîtes contrôle a permis d'estimer le nombre de cfu à 27 000 par boîte pour le mutant.

### 2.3.2. Sélection des clones

L'évènement de recombinaison, c'est-à-dire le remplacement du gène *lpt*3 TR (tronqué) par le gène *lpt*3 FL (complet), a été détecté après transfert des clones sur membrane d'hybridation et hybridation avec une sonde marquée au ³³P dCTP selon les méthodes décrites dans les sections A.1.2. et A.1.3.

La sélection des clones positifs s'est faite par hybridation de l'ADN fixé sur les membranes avec une sonde ADN marquée au ³³P dCTP correspondant à la partie tronquée du gène *lpt3,* donc présente uniquement chez les clones recombinants.

### Préparation de la sonde

Afin d'obtenir la sonde *lpt*3 de 270 pb, 10 ng du plasmide pUC*lpt*3 ont été utilisés pour amplification par PCR avec la Platinum® *Taq* DNA polymerase High Fidelity (Invitrogen, #11304-011).

Le couple d'amorces est le suivant (couple n°2) :
CGC CGA ATA CTT TAT CTT GAG GC (Tm = 60,6°C) ; et
CTC GCC AAA GAG CAG GGC (Tm = 60,5°C).

Pour amplification, le mélange suivant a été réalisé :

| Composants | Volume | Concentration finale |
|---|---|---|
| Tampon 10X High Fidelity PCR | 5 µl | 1X |
| Mélange 10 mM dNTP | 1 µl | 0.2 mM de chaque |
| MgSO₄ 50 mM | 2 µl | 2 mM |
| Mélange d'amorces (10 µM chacune) | 1 µl | 0.2 µM de chaque |
| Plasmide pUC | x µl | 100 ng |
| Platinum® *Taq* High Fidelity | 0.2 µl | 1.0 unit |
| Eau dépourvue de nucléase | pour 50 µl final | Ne s'applique pas |

Le programme du thermocycleur est le suivant :

| | | |
|---|---|---|
| Dénaturation initiale : | | 94°C pendant 30 secondes |
| 30 cycles de : | Dénaturation : | 94°C pendant 30 secondes |
| | Hybridation : | 55°C pendant 30 secondes |
| | Extension : | 68°C pendant 45 secondes. |

Après la réaction, 1/10 des produits PCR a été déposé sur gel d'agarose pour s'assurer de la spécificité de l'amplicon, puis le fragment PCR a été purifié en utilisant le kit QIAquick PCR Purification Kit (Qiagen, #28104).

### Hybridation et révélation

Les étapes de marquage de la sonde, d'hybridation, de lavages et de révélation ont été réalisées selon ce qui est décrit dans la section A.1.3.

Environ 460 000 cfu ont été testées. Après mise en exposition avec les films BioMax MR, les autoradiographies ont révélé 5 spots positifs (C708 contenant un gène *lpt*3 FL) chacun sur une membrane différente.

### Criblage et authentification des clones positifs

Après repérage sur la boîte de Pétri, une partie de la zone prélevée autour des clones positifs a été conservée en milieu de congélation (milieu M199, sérum de veau foetal 20 %, glycérol 10 %) et l'autre partie a été utilisée pour l'authentification par PCR.

Pour ce faire, chacun des prélèvements a d'abord été repris par 80 µl de bouillon BHI, de façon à étaler 30 µl de cette suspension, en mini nappe sur une boîte BHI.

Le volume restant a été centrifugé 5 min à 6000 rpm, puis le culot a été repris par 50 µl d'eau dépourvue de nucléase. Les germes ont été lysés 5 min à 95°C et le surnageant, qui sert de matrice à la réaction PCR, a été prélevé après centrifugation.

Pour chaque prélèvement correspondant à un spot positif ainsi que les témoins, une amplification PCR avec le couple d'amorces ayant servi à l'amplification de la sonde C708 *lpt*3 de 270 pb (couple n°2) a été réalisée avec le kit Expand Long Template PCR (Roche) comme décrit ci-dessous.

| Composants | Volume | Concentration Finale |
|---|---|---|
| Tampon 10X ELT PCR | 5 µl | 1X |
| Mélange de dNTP (10 mM de chaque) | 2 µl | 0.4 mM de chaque |
| Mélange d'amorces (10 µM de chaque) | 1,5 µl | 0.3 µM de chaque |
| Matrice d'ADN | 40 µl | Ne s'applique pas |
| Polymerase ELT | 0.75 µl | 3.75 unités |
| Eau dépourvue de nucléase | pour 50 µl | Ne s'applique pas |

Le programme du thermocycleur est le suivant :

| | | |
|---|---|---|
| Dénaturation initiale : | | 94°C pendant 2 min |
| 10 cycles de: | Dénaturation: | 94°C pendant 10 secondes |
| | Hybridation : | 54°C pendant 30 secondes |
| | Extension : | 68°C pendant 45 secondes |
| 20 cycles de : | Dénaturation : | 94°C pendant 15 secondes |
| | Hybridation : | 54°C pendant 30 secondes |
| | Extension : | 68°C pendant 45 secondes + 20 sec/cycle |
| Elongation finale : | | 68°C pendant 7 min |

Après la réaction, 1/10 des produits PCR a été déposé sur gel d'agarose pour vérification. Quatre des 5 clones présentaient le profil attendu. La fréquence d'obtention d'un clone positif vrai était de 1/ 115 000 cfu testées.

L'étape suivante a consisté à isoler un clone pur. Pour cela, un des clones positifs hétérogènes a été étalé en cfu isolées et plusieurs de ces cfu (40) ont été analysées en PCR, avec les couples d'amorces 1 ou 2.

Chaque cfu a été resuspendue dans 100 µl d'eau dépourvue de nucléase, 30 µl ont été déposés sur une boîte BHI et les 70 µl restants ont été lysés 5 min à 95°C et le surnageant, qui sert de matrice à la réaction PCR, a été prélevé après centrifugation.

Les PCR ont été réalisées avec la Platinum® *Taq* High Fidelity (Invitrogen) comme déjà décrit pour l'amplification de la sonde *lpt*3. La température d'hybridation était de 54°C.

Après la réaction, 1/10 des produits PCR a été déposé sur gel d'agarose pour vérification. Cinq des 40 clones se sont révélés être des clones purs.

La mini nappe des clones purs a été reprise en milieu de congélation, aliquotée sous 100 µl et conservée à -70°C. La pureté et l'identité de ce congelât ont été validées.

### 3. Construction d'une souche de N. meningitidis exprimant un LOS dont la chaîne α est celle d'un LOS d'immunotype L6 et comportant uniquement en position 03 du résidu heptose II (hep II) du core interne, un substituant phosphoéthanolamine (PEA)

On utilise pour souche de départ, la souche de *N. meningitidis* C708 *lpt*3 FL obtenue comme précédemment décrit. L'objectif est d'inactiver le gène *lpt*6 de cette souche par délétion d'une partie centrale du gène.

### 3.1. Amplification par PCR (polymerase chain reaction) du gène lpt6 complet (full-length) de la souche de N. meningitidis Z2491 de sérogroupe A (gène NMA 0408)

Cent ng d'ADN génomique de la souche Z2491 (souche mise à disposition dans les laboratoires de recherche, à l'échelle mondiale) ont été utilisés pour amplification avec la Platinum® *Taq* DNA polymerase High Fidelity (Invitrogen, #11304-011).

Le couple d'amorces est le suivant (couple n°3) :
CG GAATTC GCC GTC TCA A GGT TGC CTA TGT TTT CCT GTT TTT G (Tm = 59,7°C) ; et
AA CTGCAG CTA ACG GGC AAT TTT CAA AAC GTC (Tm = 59,3°C). (sont respectivement soulignés les sites *Eco*RI et *Pst*I).

Pour amplification le mélange suivant a été réalisé :

| Composants | Volume | Concentration finale |
|---|---|---|
| Tampon 10X High Fidelity PCR | 5 µl | 1X |
| Mélange 10 mM dNTP | 1 µl | 0.2 mM de chaque |
| MgSO₄ 50 mM | 2 µl | 2 mM |
| Mélange d'amorces (10 µM chacune) | 1 µl | 0.2 µM de chaque |
| ADN génomique | x µl | 100 ng |
| Platinum® *Taq* High Fidelity | 0.2 µl | 1.0 unit |
| Eau dépourvue de nucléase | pour 50 µl final | Ne s'applique pas |

Le programme du thermocycleur est le suivant :

| | | |
|---|---|---|
| Dénaturation initiale : | | 94°C pendant 30 secondes |
| 30 cycles de : | Dénaturation : | 94°C pendant 30 secondes |
| | Hybridation : | 55°C pendant 30 secondes |
| | Extension : | 68°C pendant 1 minute / kb de produit PCR. |

Après la réaction, 1/10 du produit PCR a été déposé sur gel d'agarose pour vérification.

### 3.2. Construction du vecteur pM1223 (pUC19 lpt6 FL)

Le produit PCR d'une part et le plasmide pUC19 d'autre part ont été soumis à une double digestion par *Eco*RI et *Pst*I pendant 2 hrs à 37°C. On a utilisé 10 unités de chaque enzyme par µg d'ADN dans le tampon REact2 (Invitrogen).

Le fragment PCR a été ensuite inséré dans le vecteur pUC19 linéarisé. Les ligations ont été réalisées sous un volume final de 20 µl avec 50 ng de vecteur, 0,5 U de T4 DNA ligase (Invitrogen) et 1 µl d'ATP 10 mM (Invitrogen) pendant 16 heures à 16°C. La ligase a ensuite été inactivée par chauffage 10 min à 65°C.

Le vecteur ainsi obtenu a été transféré par la technique d'électroporation dans une souche d'*E. coli* XL1 blue MRF résistante à la kanamycine et rendue électro compétente. Les paramètres suivis pour l'électroporation sont les suivants : Capacitance : 500 µFD ; Résistance : 200 ohms ; Voltage : 1700 volts.

La sélection des clones transformés a été faite par étalement sur boîtes LB ampicilline 100 µg/ml. L'authentification des clones positifs (présence d'un gène *lpt*6 FL) a été réalisée par digestion enzymatique *Nde*I après extraction de l'ADN par miniprep. Sur 20 clones analysés, 6 présentaient le profil attendu. Le plasmide recombinant du clone sélectionné a été nommé pM1223.

### 3.3. Délétion de la partie centrale du gène lpt6 provenant de la souche Z2491

### Construction d'un vecteur de transformation

Avec le kit Expand Long Template PCR (Roche), on a réalisé une PCR inverse à partir du plasmide pM1223 à l'aide du couple d'amorces suivant (couple n°4) :
CG GGATCC CAT CGA CAC GAA CGC CGC (Tm = 60,5); et
CG GGATCC CCG CGC TTA ACG ACT ACA TC (Tm = 59,4) ;
   (sont soulignés les sites *Bam*HI).

Ceci permet de réamplifier le plasmide en délétant la partie que l'on souhaite retirer (808 bp).

Pour amplification le mélange suivant a été réalisé :

| Composants | Volume | Concentration Finale |
|---|---|---|
| Tampon 10X ELT PCR | 5 µl | 1X |
| Mélange de dNTP (10 mM de chaque) | 2 µl | 0.4 mM de chaque |
| Mélange d'amorces (10 µM de chaque) | 1,5 µl | 0.3 µM de chaque |
| Matrice d'ADN (pM1223) | 40 µl | Ne s'applique pas |
| Polymérase ELT | 0.75 µl | 3.75 unités |
| Eau dépourvue de nucléase | pour 50 µl | Ne s'applique pas |

Le programme du thermocycleur est le suivant :

| | | |
|---|---|---|
| Dénaturation initiale : | | 94°C pendant 2 min |
| 10 cycles de : | Dénaturation : | 94°C pendant 10 secondes |
| | Hybridation : | 54°C pendant 30 secondes |
| | Extension : | 68°C pendant 3 min |
| 20 cycles de : | Dénaturation : | 94°C pendant 15 secondes |
| | Hybridation : | 54°C pendant 30 secondes |
| | Extension : | 68°C pendant 3 min + 20 sec/cycle |
| Elongation finale : | | 68°C pendant 7 min |

Après la réaction, 1/10 des produits PCR a été déposé sur gel d'agarose.

Après purification sur colonne QiaQuick, le produit PCR a été digéré par *Bam*HI à raison de 10 U d'enzyme par µg d'ADN. Une fois digéré, il a été purifié par électroélution puis extraction phénol-chloroforme.

La ligation du vecteur sur lui-même a été réalisée sous un volume final de 20 µl avec 0,5 U de T4 DNA ligase (Invitrogen) et 1 µl d'ATP 10 mM (Invitrogen) pendant 16 heures à 16°C. La ligase a ensuite été inactivée par chauffage 10 min à 65°C.

La dernière étape a consisté à transférer le vecteur ainsi ligué dans une souche d'*Escherichia coli* comme décrit pour le pM1222. L'authentification de clones positifs a été réalisée par digestion enzymatique *Nde*I-*Pst*I après extraction de l'ADN par miniprep. Sur les 4 clones analysés 100 % présentent le profil attendu.

Le plasmide recombinant du clone positif sélectionné a été renommé pM1224 et ce clone a été conservé en glycérol à -70°C. La présence dans le plasmide pM1224 d'un gène *lgt*6 délété de sa partie centrale a été vérifiée par séquençage.

### 3.4. Transformation de la souche C708 lpt3 FL et détection de l'événement de recombinaison homologue

### Transformation

Dix µg de plasmide pM1224 ont été linéarisés par *Eco*RI à raison de 10 unités d'enzyme par µg de plasmide à digérer dans le tampon approprié pendant 2 heures à 37°C.

La transformation dans la souche C708 a été faite en suivant la technique décrite dans la section A.1.1.

Après transformation, les bactéries ont été étalées sur 16 boîtes de Pétri de 140 mm à une concentration théorique de 50 000 cfu par boîte ; soit 800 000 cfu. Les boîtes ont été placées une nuit à 37°C puis ensuite été placées 30 min à +4°C.

### Sélection des clones : Préparation de la sonde, hybridation et révélation

L'évènement de recombinaison a été détecté après colony blot et hybridation avec un oligonucléotide marquée au γ³²P dATP.

L'évènement de recombinaison, c'est-à-dire le remplacement du gène *lpt6* FL par le gène *lpt*6 TR, a été détecté après transfert des clones sur membrane et hybridation avec une sonde marquée selon les méthodes décrites dans les sections A.1.2. et A.1.4.

Les clones transférés sur membranes sont soumis à des étapes de lyse et de lavage. L'ADN est fixé sur les membranes en plaçant ces dernières 2 heures à 80°C.

La sélection des clones positifs s'est faite par hybridation de l'ADN fixé sur membranes Hybond N+ avec un oligonucléotide radioactif dont la séquence est chevauchante sur les deux extrémités recombigéniques. Il s'agit de l'oligonucléotide suivant : GTC GAT GGG ATC CCC GCG CTT AAC G (Tm = 69.5°C).

Environ 840 000 cfu ont été testées. Après mise en exposition avec les films BioMax MR, les autoradiographies ont révélé 16 spots positifs (C708 contenant un gène *lpt*6 TR) répartis sur 9 membranes différentes.

### Criblage et authentification des clones positifs

Pour chacun des 16 spots positifs, après repérage sur la boîte de Pétri, une partie de la zone prélevée autour des clones positifs a été conservée en milieu de congélation (M199, SVF 20 %, glycérol 10 %) et l'autre partie a été utilisée pour l'authentification par PCR.

Pour ce faire les prélèvements ont d'abord été repris par 80 µl de bouillon BHI, de façon à étaler, en mini nappe sur une boîte BHI, 30 µl de chaque suspension.

Le volume restant a été centrifugé 5 min à 6000 rpm, puis le culot a été repris par 50 µl d'eau dépourvue de nucléase. Les germes ont été lysés 5 min à 95°C et le surnageant, qui sert de matrice à la réaction PCR, a été prélevé après centrifugation.

Pour chaque prélèvement correspondant à un spot positif, une amplification PCR a été réalisée avec la Platinum® *Taq* High Fidelity (Invitrogen) et le couple d'amorces suivant (couple n°5)
CCG ACT GGC GGA ATT GGG (TM = 60,5°C) ; et
CCC ATT TCT TCC TGA CGG AC (Tm = 59,4°C).

Pour amplification, le mélange suivant a été réalisé :

| Composants | Volume | Concentration finale |
|---|---|---|
| Tampon 10X High Fidelity PCR | 5 µl | 1X |
| Mélange 10 mM dNTP | 1 µl | 0.2 mM de chaque |
| MgSO₄ 50 mM | 2 µl | 2 mM |
| Mélange d'amorces (10 µM chacune) | 1 µl | 0.2 µM de chaque |
| Matrice d'ADN | x µl | 100 ng |
| Platinum® *Taq* High Fidelity | 0.2 µl | 1.0 unit |
| Eau dépourvue de nucléase | pour 50 µl final | Ne s'applique pas |

Le programme du thermocycleur est le suivant :

| | | |
|---|---|---|
| Dénaturation initiale : | | 94°C pendant 1 min |
| 30 cycles de : | Dénaturation : | 94°C pendant 30 secondes |
| | Hybridation : | 55°C pendant 30 secondes |
| | Extension : | 68°C pendant 50 secondes. |

Après la réaction, 1/10 du produit PCR a été déposé sur gel d'agarose, pour vérification. Deux candidats sur 16 se sont révélés être de vrais positifs : soit une fréquence d'obtention d'un clone positif vrai pour 425 000 cfu testées.

L'étape suivante a consisté à isoler un clone pur. Pour cela, un des 2 clones positifs hétérogènes a été étalé en cfu isolées et plusieurs de ces cfu (24) ont été analysées en PCR, avec le couple d'amorces n°5.

Chaque cfu a été resuspendue dans 50 µl d'eau dépourvue de nucléase, 20 µl ont été déposés sur une boîte BHI et les 30 µl restant ont été lysés 5 min à 95°C et le surnageant, qui sert de matrice à la réaction PCR, a été prélevé après centrifugation.

Les PCR ont été réalisées avec la Platinum® *Taq* High Fidelity (Invitrogen) comme déjà décrit pour le criblage des prélèvements des spots positifs.

Après la réaction, 1/5 des produits PCR a été déposé sur gel d'agarose pour vérification. Seul un clone sur 24 s'est révélé être un positif vrai.

La mini nappe du clone positif pur a été reprise en milieu de congélation, aliquotée sous 100 µl et conservée à -70°C. La pureté et identité de ce congelât ont été validées.

### 4. Construction d'une souche de N. meningitidis exprimant un LOS dont la chaîne α est celle d'un LOS d'immunotype L8 et comportant uniquement en position 03 du résidu heptose II (hep II) du core interne, un substituant phosphoéthanolamine (PEA)

On utilise pour souche de départ, la souche de *N*. *meningitidis* C708 *lpt*3 FL *lpt*6 TR obtenue comme précédemment décrit dans la section A.3. L'objectif est d'inactiver le gène *lgtA* de cette souche par délétion d'une partie centrale du gène.

### 4.1. Amplification par PCR (polymerase chain reaction) du gène lgt A complet (full-length) de la souche de N. meningitidis MC58 de sérogroupe B (gène NMB 1929)

Cent ng d'ADN génomique de la souche MC58 (souche mise à disposition dans les laboratoires de recherche, à l'échelle mondiale) ont été utilisés pour amplification avec la Platinum® *Taq* DNA polymerase High Fidelity (Invitrogen, #11304-011).

Le couple d'amorces est le suivant :
CG GAATTC GCC GTC TCA A ATG CCG TCT GAA GCC TTC AG (Tm = 59,4°C) ; et
AA CTGCAG AAC GGT TTT TCA GCA ATC GGT GC (Tm = 60,6°C).
   (sont respectivement soulignés les sites *Eco*RI et *Pst*I).

Pour amplification le mélange suivant a été réalisé :

| Composants | Volume | Concentration finale |
|---|---|---|
| Tampon 10X High Fidelity PCR | 5 µl | 1X |
| Mélange 10 mM dNTP | 1 µl | 0.2 mM de chaque |
| MgSO₄ 50 mM | 2 µl | 2 mM |
| Mélange d'amorces (10 µM chacune) | 1 µl | 0.2 µM de chaque |
| ADN génomique | x µl | 100 ng |
| Platinum® *Taq* High Fidelity | 0.2 µl | 1.0 unit |
| Eau dépourvue de nucléase | pour 50 µl final | Ne s'applique pas |

Le programme du thermocycleur est le suivant :

| | | |
|---|---|---|
| Dénaturation initiale : | | 94°C pendant 30 secondes |
| 30 cycles de : | Dénaturation : | 94°C pendant 30 secondes |
| | Hybridation : | 55°C pendant 30 secondes |
| | Extension : | 68°C pendant 1 minute / kb de produit PCR. |

Après la réaction, 1/10 du produit PCR a été déposé sur gel d'agarose pour vérification.

### 4.2. Construction du vecteur pUC19 lgtA FL

Le produit PCR obtenu en 4.1. d'une part et le plasmide pUC19 d'autre part ont été soumis à une double digestion par *Eco*RI et *Pst*I pendant 2 hrs à 37°C. On a utilisé 10 unités de chaque enzyme par µg d'ADN dans le tampon REact2 (Invitrogen).

Le fragment PCR a été ensuite inséré dans le vecteur pUC19 linéarisé. Les ligations ont été réalisées sous un volume final de 20 µl avec 50 ng de vecteur, 0,5 U de T4 DNA ligase (Invitrogen) et 1 µl d'ATP 10 mM (Invitrogen) pendant 16 heures à 16°C. La ligase a ensuite été inactivée par chauffage 10 min à 65°C.

Le vecteur ainsi obtenu a été transféré par la technique d'électroporation dans la souche d'*E. coli* XL1 blue MRF résistante à la kanamycine et rendue électro-compétente. Les paramètres suivis pour l'électroporation sont les suivants : Capacitance : 500 µFD ; Résistance : 200 ohms ; Voltage : 1700 volts.

La sélection des clones transformés a été faite par étalement sur boîtes LB ampicilline 100 µg/ml. L'authentification des clones positifs (présence d'un gène *lgt*A FL) a été réalisée par digestion enzymatique après extraction de l'ADN par miniprep. 1/5 des clones analysés présentaient le profil de digestion enzymatique attendu.

### 4.3. Amplification par PCR (polymerase chain reaction) du gène de résistance à l'érythromycine (erm)

L'amplification par PCR de la cassette érythromycine (erm) au départ du pMGC10 a été faite avec des amorces permettant d'intégrer les sites de restriction *Bam*HI-*Xba*I.

Le couple d'amorces utilisé est le suivant :
CG GGATCC GGA AGG CCC GAG CGC AGA AGT (Tm : 65,7°C) ; et
GC TCTAGA CAA CTT ACT TCT GAC AAC GAT CGG (Tm : 61 °C)

Pour amplification, le mélange suivant a été réalisé :

| Composants | Volume | Concentration finale |
|---|---|---|
| Tampon Pfu turbo 10X | 5 µl | 1X |
| 10 mM dNTP mixture | 0.4 µl | 0.2 mM de chaque |
| Mélange d'amorces (10 µM chacune) | 1 µl | 0.2 |
| pMGC10 | x µl | 10 ng |
| Pfu turbo (Stratagène) | 1 µl | 2.5 units |
| Nuclease free water | to 50 µl | Pas applicable |

Le programme du thermocycleur a été le suivant:
Initial denaturation: 95°C for 2 mn
30 cycles of:
   Denature: 95°C for 30 seconds
   Anneal: 55°C for 30 seconds
   Extend: 72°C for 1 minute
   Final elongation: 72°C for 10 mn

Après la réaction 1/10 des produits PCR sont déposés sur gel d'agarose.

### 4.4. Construction du plasmide pUC19 lgtA TR (délétion de la partie centrale du gène lgtA par PCR inverse)

Avec le kit Expand Long Template PCR (Roche), on a réalisé une PCR inverse à partir du plasmide pUC19 *lgt*A FL, obtenu en A.4.2., dans le double but d'enlever la partie centrale du gène et de créer deux sites de restriction aux extrémités (*Bam*HI et *Xba*I). Le couple d'amorces suivant a été utilisé :
CG GGATCC GCC AAT TCA TCC AGC CCG ATG (Tm = 61,8°C); et
CG TCTAGA CCC GGT TCG ACA GCC TTG (Tm = 60,5°C) ;
   Ceci permet de réamplifier le plasmide en délétant la partie que l'on souhaite retirer.

Pour amplification, le mélange suivant a été réalisé :

| Composants | Volume | Concentration Finale |
|---|---|---|
| Tampon 10X ELT PCR | 5 µl | 1X |
| Mélange de dNTP (10 mM de chaque) | 2 µl | 0.4 mM de chaque |
| Mélange d'amorces (10 µM de chaque) | 1,5 µl | 0.3 µM de chaque |
| Matrice d'ADN (10 ng de pUC19 *lgt*A FL) | | Ne s'applique pas |
| Polymérase ELT | 0.75 µl | 3.75 unités |
| Eau dépourvue de nucléase | pour 50 µl | Ne s'applique pas |

Le programme du thermocycleur est le suivant :

| | | |
|---|---|---|
| Dénaturation initiale : | | 94°C pendant 2 min |
| 10 cycles de : | Dénaturation : | 94°C pendant 10 secondes |
| | Hybridation : | 55°C pendant 30 secondes |
| | Extension : | 68°C pendant 1 min par kbs |
| 20 cycles de : | Dénaturation : | 94°C pendant 15 secondes |
| | Hybridation : | 55°C pendant 30 secondes |
| | Extension : | 68°C pendant 1 min par kbs + 20 sec/cycle |
| Elongation finale : | | 68°C pendant 7 min |

Après la réaction, 1/10 des produits PCR a été déposé sur gel d'agarose pour vérification de la taille (3.2.kbs). Le produit PCR a été purifié sur colonne QiaQuick.

La dernière étape a consisté à transférer le vecteur dans la souche d'*E. coli* XL1 blue MRF résistante à la kanamycine et rendue électro compétente. L'authentification de clones positifs (*lgt*A délété de sa partie centrale) a été réalisée par digestion enzymatique après extraction de l'ADN par miniprep.

### 4.5. Construction du plasmide pUC19 lgtA :: erm

Le produit PCR erm d'une part et le plasmide pUC19 *lgt*A TR issu de la PCR inverse d'autre part ont été soumis chacun à une double digestion par *Bam*HI et *Xba*I dans les conditions suivantes :
2 µg d'ADN ont été en contact avec 20 unités de *Xba*I en tampon 2 (InVitrogen) sous 60 µl pendant 2 heures à 37°C. Puis *Xba*I a été inactivée 10 minutes à 65°C. On a alors ajouté 7 µl de NaCl 1 M, 20 unités de *Bam*HI et 1 µl de tampon 2. La digestion a été poursuivie 2 hrs à 37°C.
Les produits de digestion ont ensuite été intégralement déposés sur un gel d'agarose 0.8%, et après migration, les bandes ont été découpées pour être électroéluées (celle du plasmide est à 3.2 kbs).

Après purification, le plasmide linearisé et le produit PCR erm digéré ont été ligués entre eux comme précédemment décrit. Le produit de la ligation a été utilisé pour transformer comme précédemment décrit, la souche *E*. *coli* XL1 Blue MRF résistante à la kanamycine et rendue électro-compétente. Les clones recombinants ont été analysés par digestion enzymatique. 4/11 des clones analysés présentaient le profil de digestion enzymatique attendu.

### 4.6. Transformation de la souche C708 lpt3 FL lpt6 TR et détection de l'évènement de recombinaison homologue

Dix µg de plasmide pUC19 *lgt*A :: erm ont été linéarisés par *Eco*RI à raison de 10 unités d'enzyme par µg de plasmide à digérer dans le tampon approprié pendant 2 heures à 37°C.

La transformation dans la souche C708 *lpt*3 FL *lpt*6 TR a été faite en suivant la technique décrite dans la section A.1.1.

Après transformation, 1,24 10⁸ bactéries ont été étalées sur milieu BHI + erythromycine à 2 µg/mL. L'incubation est poursuivie une nuit à 37°C. La fréquence de transformation observée est de 1 / 2,5 10⁶.

### B. Données expérimentales relatives aux compositions vaccinales

### 1. Préparation de la rTbpB lipidée

Par souci de simplification langagière, on indiquera simplement par la suite, rTbpB ou TbpB.

### 1.1. Production

### Souches exprimant la TbpB lipidée M982 ou B16B6

Les souches d'expression sont les souches d'*E*. *coli* BL21 contenant respectivement le plasmide pTG9219 / pTG9216. Ces plasmides comportent notamment un marqueur de sélection à la kanamycine et le polynucléotide codant pour la rTbpB de la souche de *N. meningitidis* M982 (pTG9219) ou B16B6 (pTG9216) (les séquences sont telles que décrites dans le brevet EP 586 266), fusionnée à la séquence signal R1pB (Real lipoprotein B) *d'E. coli* et placée sous le contrôle du promoteur arabinose (*ara*B)*.*

### Culture

Trois congelâts de la souche d*'E. coli* BL21/pTG9219 ou BL21/pTG9216 (1 ml chacun) servent à ensemencer 3 litres de milieu LB (Luria Broth) répartis en erlens. L'incubation est poursuivie 15 à 18 h à 37°C.

Cette pré-culture sert à ensemencer un fermenteur contenant du milieu TGM16 (extrait de levure 9g/L, K₂SO₄ 0.795 g/L, K₂HPO₄ 3.15 g/L, NaCl 0.75 g/L, CaCl₂,2H₂O 0.005 g/L, FeCl₃,6H₂O 0.021 g/L, MgSO₄,7H₂O 0.69 g/L, hydrolysat acide de caséine sans sel 37.5 g/L) supplémenté avec du glycérol 20 g/L, à raison de 10 % (vol./vol).

La culture est poursuivie à 37°C sous agitation, à une pression de 100 mbar et sous une alimentation d'air de 1 L/min/L de culture, en réajustant au cours du temps la concentration de glycérol à 20 g/L (*e.g.* à DO₆₀₀ de 15 ± 2). Lorsque la DO₆₀₀ est comprise entre 21 et 27, on induit l'expression de la rTbpB par addition d'arabinose pour obtenir une concentration finale de 10 g/L. Après une heure d'induction, la culture est arrêtée en-refroidissant aux alentours de 10°C.

Les culots bactériens sont récupérés par centrifugation et stockés au froid.

### 1.2. Purification

### Extraction des membranes renfermant la rTbpB

### LOS extraction

Un culot bactérien équivalent à un litre de culture (environ 72 g de germes, poids humide) est décongelé à une température de 20°C +/-5°C. Les germes décongelés (ou partiellement décongelés) sont remis en suspension par 800 ml d'une solution à température ambiante de Tris HCl 50 mM, EDTA 5 mM, pH 8,0. On ajoute aussitôt 9 pastilles d'inhibiteur de protéases (7 pastilles de Complete Mini, EDTA free ; ROCHE réf. 11836170001 + deux pastilles de Complete, EDTA free; ROCHE réf. 11836170001). Une partie des germes se lysant spontanément, 4 µl de benzonase (1 UI d'activité DNAse/ml en final ; Merck réf K32475095) sont ajoutés également. L'incubation est poursuivie à +4°C durant 45 minutes sous agitation magnétique après homogénéisation par Turrax (15 sec.).

Puis on ajoute 4 ml de MgCl₂ 1 M afin d'être à 5 mM final. L'agitation magnétique est portée à 10 minutes. Une centrifugation à 15,000 g durant 45 minutes permet de récolter le culot (culot C1 ; *vs* Surnageant S1) contenant la protéine rTbpB.

Une deuxième extraction est réalisée : homogénéisation par Turrax dans 800 ml du tampon Tris HCl 50 mM, EDTA 5 mM pH 8,0 et agitation pendant 30 min. On ajoute du MgCl₂ (8 ml d'une solution molaire). L'incubation est poursuivie pendant 10 minutes. La suspension est centrifugée 15,000 g durant 1 hr 30.

### Lyse bactérienne

Le culot est remis en suspension par 1400 ml de Tris HCl 50 mM additionné de 4 pastilles d'inhibiteur de protéases et de 8 µl de Benzonase. La solution est homogénéisée au Turrax 15 secondes. La lyse est réalisée à +4°C durant 30 minutes grâce à l'ajout de 14 ml (10 mg/ml en final) de lysozyme à 100 mg/ml en acétate de Na 25 mM, glycérol 50%.

La suspension est centrifugée à 30,000 g durant 30 minutes (Culot C2 contenant la protéine ; *vs* surnageant S2 contenant les contaminants de rTbpB). Le culot contenant les membranes peut être congelé à ce stade.

### Lavage des fragments de membranes

Le culot de lyse C2 est repris en Tris HCl 50 mM (1100 ml). Après homogénéisation, (Turrax 15 secondes), il est lavé durant une heure à +4°C. On opère comme précédemment à une centrifugation à 30,000 g durant 30 minutes, Le culot (C3 ; *vs* surnageant S3) est congelé à -45°C. Le tampon Tris HCl 50 mM permet d'éliminer une petite quantité de protéine (surnageant S3) et ne solubilise que très peu de rTbpB.

Le culot C3 est repris en tampon Tris HCl 50 mM, urée 8 M pH 8,0 (800 ml). Ce tampon permet d'éliminer une partie des protéines contaminantes sans solubiliser les membranes contenant la rTbpB. Après homogénéisation (sans Turrax), la solution est alors agitée durant une heure à +4°C. On procède comme précédemment à une centrifugation à 30,000 g durant 30 minutes qui permet d'obtenir un culot de membranes qui peut être congelé.

### Solubilisation des membranes

Le culot de membrane décongelé est solubilisé par 780 ml de tampon Tris HCl 50 mM EDTA 6 mM Urée 2 M Elugent 4 % à pH 7,5. La présence du détergent à 4 % et de l'urée 2 M permet de réaliser la solubilisation du culot. La solution est agitée à +4°C une nuit (16h minimum). La centrifugation à 30,000 g (1 heure à +4°C) de la solution ne laisse qu'un petit culot (C4) contenant quelques impuretés. Le surnageant S4 contenant la protéine rTbpB est récupéré pour dépôt sur une première colonne échangeuse de cations (QS I).

### Purification par chromatographie échangeuse d'anions sur Q Sepharose à pH 7,5

Deux chromatographies successives sont réalisées, le produit de la première chromatographie est collecté puis déposé ensuite après une étape de dialyse sur une deuxième colonne de chromatographie qui utilise des conditions différentes (absence d'EDTA).

### 1^{ère} Chromatographie, en présence d EDTA (Chromatographie QS I)

Une colonne de 600 ml (K50, diamètre 20 cm2) de gel Q Sepharose Fast Flow (réf 17-0510-01 GE Healthcare) est montée, tassée en tampon d'équilibration Tris HCl 50 mM EDTA 6 mM, Urée 2 M, Elugent 1 %, à pH 7,5 au débit de 8 ml/minute.

Le surnageant S4 (environ 845 ml) est déposé au débit de 6 ml/minute. L'éluât direct (partie qui ne s'accroche pas sur la colonne lors du dépôt de l'échantillon) contient la protéine d'intérêt rTbpB. Cet éluât (1150 mL) est prélevé, puis dialysé à +4°C (durant 6 jours) contre 6 litres de tampon Tris HCl 50 mM, Urée 2 M, Elugent 1 %, pH 7,5 afin d'abaisser à 1 mM la concentration en EDTA et d'éliminer le NaCl.

### 2^{éme} chromatographie (QS II), sans EDTA

Une colonne K50 de 490 ml de gel neuf Q Sepharose Fast Flow est équilibrée en tampon Tris HCl 50 mM, Urée 2 M, Elugent 1 % pH 7,5.

La solution dialysée (1080 ml) est déposée sur la colonne (débit 6 ml/minute) ; puis 5 paliers d'élution salins dans ce même tampon sont réalisés: 20 mM, 50 mM, 100 mM, 250 mM et 1 M NaCl (débit de travail 6 ml/minute). La protéine rTbpB est éluée de la colonne à deux concentrations en sel (50 mM et 100 mM). La fraction d'élution 50 mM est la fraction d'intérêt, la protéine rTbpB y étant la plus pure et en plus importante quantité (2,6 fois plus de protéine que dans la fraction 100 mM de NaCl)

Le pH de la fraction correspondant au pic d'élution 50 mM NaCl est abaissé sous agitation magnétique à pH 5,5 par un ajout d'acide acétique 1,7 N. La solution (860 ml) est placée en dialyse contre 5 litres de tampon acétate de sodium 10 mM, Urée 1 M, Elugent 0,2 %, pH 5,5 (24 hrs à +4°C) puis contre 4 litres de tampon acétate de sodium 10 mM, Urée 1 M, Elugent 0,2 %, pH 5,5 (17 hrs à +4°C).

### Purification par chromatographie échangeuse de cations sur SP Sepharose (SPI) à pH 5,5

Une colonne K50 de 100 ml de gel neuf SP Sepharose Fast Flow (Ge Healthcare, réf 17-0729-01) est équilibrée en tampon acétate de sodium 10 mM, Urée 1 M, Elugent 0,2 %, pH 5,5.

La solution de protéine dialysée (850 ml) est déposée sur la colonne (débit 6 ml/minute). Puis, cinq paliers salins d'élution sont réalisés: 50 mM, 100 mM, 250 mM, 500 mM et 1 M NaCl, en tampon cité ci-dessus.

La protéine rTbpB est éluée exclusivement dans la fraction 250 mM NaCl et les contaminants de faible poids moléculaire sont éliminés essentiellement dans l'éluât direct (40 %). On obtient ainsi environ 35 mg de rTbpB M982 purifiée et un peu moins pour ce qui concerne la rTbpB B16B6.

### Dialyse et concentration du produit de SPI (fractions 250 mM)

Les fractions correspondant au pic d'élution 250 mM de la colonne SPI sont rassemblés (volume 274 ml). Le pH de la solution est remonté à pH 7,3 par ajout sous agitation d'environ 800 µl de NaOH 0,5 N. La solution est placée en dialyse à +4°C (Spectra Por 1 : seuil de coupure 6-8000 D) contre deux bains de 10 litres de PBS Elugent 0,2 % pH 7,1 (66 hrs et 22 hrs).

Le dialysat est concentré jusqu'à un volume de 21,1 ml par diafiltration concentration frontale sur membrane Amicon 30 kD en PBS (réf PBTK06510).

Puis le concentré obtenu est remis en dialyse contre 2 litres de PBS Elugent 0,2 % pH 7,1 (Slide A Lyser réf 66810: seuil de coupure 10 kD).

La solution est ensuite filtrée de façon aseptique sur filtre Millex 0,22 µm en Durapore (réf Millipore SLGV 033RS). Le lot de protéine rTbpB purifiée obtenu est congelé à - 80°C. La concentration en protéine est de 1642 µg/ml.

### 1.3. Préparation de la rTbpB pour injection

La solution de rTbpB obtenue en section B.1.2. est traitée par adsorption sur des Bio-Beads™ SM-2 pour éliminer l'excès du détergent Elugent™ (tensio-actif constitué d'alkyl glucosides) qui pourrait déstabiliser les liposomes LOS.

### Activation des Bio-Beads™

On ajoute environ 2.5 mL de méthanol à 500 mg de Bio-Beads™ et on homogénéise par intermittence durant 15 min. à température ambiante. Après un temps de décantation, le surnageant est éliminé. Cette opération de lavage est renouvelée deux fois.

On ajoute alors environ 5 mL d'eau stérile ultrafiltrée et on homogénéise par intermittence durant 15 min. à température ambiante. Après un temps de décantation, le surnageant est éliminé. Cette opération de lavage est renouvelée deux fois.

On ajoute alors environ 5 mL de PBS et on homogénéise par intermittence durant 15 min. à température ambiante. On conserve à 5°C et on utilise le jour même.

En final, le poids des Bio-Beads™ s'est accru d'un facteur R (égal à environ 1.2).

### Elimination du détergent par adsorption sur Bio-Beads™

La solution de rTbpB obtenue à la section 1.2. contient 2 mg/mL d'Elugent™. La quantité de Bio-Beads™ devant être utilisée est déterminée en fonction de la quantité d'Elugent™ à éliminer.

Pour un mL de la solution de rTbpB obtenue à la section B.1.2., on ajoute 29 X R mg de Bio-Beads™ activées. On agite vivement pendant une heure à température ambiante. Puis on récupère le maximum de liquide auquel on ajoute du merthiolate 0.001 % final. Le tout est réalisé en condition stérile.

### 2. Préparation du LOS purifié

### Culture

Huit mL de congelât de l'une des souches de *N. meningitidis* C708 sérogroupe A précédemment décrites ou de la souche A1 de *N. meningitidis* sérogroupe A connue pour exprimer de manière exclusive du LOS d'immunotype L8 et dont le LOS porte 2 PEAs, l'un en position 3, l'autre en position 6 de l'heptose II, servent à ensemencer 800 mL de milieu Mueller Hinton (Merck) supplémentés avec 4 mL d'une solution de glucose à 500 g/L et répartis en erlens. La culture est poursuivie sous agitation à 36 ± 1°C pendant environ 10 heures.

On ajoute à la pré-culture 400 mL d'une solution de glucose à 500 g/L et 800 mL d'une solution d'acides aminés. Cette préparation sert à ensemencer un fermenteur contenant du milieu Mueller-Hinton, à une DO₆₀₀ₙₘ proche de 0.05. La fermentation est poursuivie à 36°C, à pH 6.8, 100 rpm, pO₂ 30 % sous un flux d'air initial de 0.75 L/min/L de culture.

Après environ 7 hrs (DO₆₀₀ₙₘ d'environ 3), on ajoute du milieu Mueller-Hinton à raison de 440 g/hr. Quand la concentration en glucose est inférieure à 5 g/L la fermentation est arrêtée. La DO₆₀₀ₙₘ finale est couramment comprise entre 20 et 40. Les cellules sont récoltées par centrifugation et les culots congelés à -35°C.

### Purification (méthode adaptée de Westphal & Jann, (1965) Meth. Carbohydr. Chem. 5 : 83)

Les culots sont décongelés et suspendus avec 3 volumes de phénol 4.5 % (vol./vol.) en agitant vigoureusement pendant 4 hrs à environ 5°C. Le LOS est extrait par traitement au phénol.

La suspension bactérienne est chauffée à 65°C puis mélangée vol./vol. avec du phénol à 90 % en agitant vigoureusement pendant 50-70 min à 65°C. La suspension est ensuite refroidie à température ambiante puis centrifugée pendant 20 min à 11 000 g. La phase aqueuse est prélevée et conservée tandis que la phase phénolique et l'interphase sont récoltées pour être soumises à une deuxième extraction.

La phase phénolique et l'interphase sont chauffées à 65°C puis mélangées à un volume d'eau équivalent à celui de la phase aqueuse précédemment prélevée en agitant vigoureusement pendant 50-70 min à 65°C. La suspension est ensuite refroidie à température ambiante puis centrifugée pendant 20 min à 11 000 g. La phase aqueuse est prélevée et conservée tandis que la phase phénolique et l'interphase sont récoltées pour être soumises à une troisième extraction identique à la seconde.

Les trois phases aqueuses sont dialysées séparément contre 40 L d'eau chacune. Puis les dialysats sont rassemblés ensemble. A 9 volumes de dialysat on ajoute un volume de Tris 20 mM, MgCl₂ 2mM. Le pH est ajusté à 8.0 ± 0.2 avec de la soude 4 N.

Deux cent cinquante unités internationales de DNAse sont ajoutées par gramme de culot. Le pH est ajusté à 6.8 ± 0.2. La préparation est placée à 37°C pendant environ 2 hrs sous agitation magnétique ; puis soumise à une filtration sur membrane de 0.22 µm. Le filtrat purifié par passage sur une colonne de Sephacryl S-300 (5.0 x 90 cm ; Pharmacia™).

Les fractions contenant le LOS sont rassemblées ensemble et la concentration en MgCl₂ est élevée à 0.5 M en rajoutant de la poudre de MgCl₂,6H₂O sous agitation.

Tout en poursuivant l'agitation, on ajoute de l'alcool absolu déshydraté pour une concentration finale de 55 % (vol./vol.). L'agitation est poursuivie une nuit à 5 ± 2°C, puis on centrifuge à 5,000 g pendant 30 min à 5 ± 2°C. Les culots sont resuspendus avec au moins 100 mL de MgCl₂ 0.5 M puis soumis à une deuxième précipitation alcoolique identique à la précédente. Les culots sont resuspendus avec au moins 100 mL de MgCl₂ 0.5 M.

La suspension est soumise à une filtration sur gel comme précédemment décrit. Les fractions contenant le LOS sont rassemblées ensemble et stérilisées par filtration (0.8-0.22 µm) et conservées à 5 ± 2°C.

Ce procédé de purification permet d'obtenir environ 150 mg de LOS par litre de culture.

### 3. Préparation des liposomes [LOS] par dialyse de détergent

### 3.1. Préparation des liposomes

Les liposomes LOS sont préparés par dialyse de détergent. En bref, les lipides (EDOPC : DOPE) sont mis sous forme de film lipidique et repris en tampon Tris 10 mM, puis dispersés en présence de 100 mM d'octyl β-D-glucopyranoside (OG) (Sigma-Aldrich ref 08001) et filtrés stérilement. Le LOS en OG 100 mM est ajouté stérilement. Le mélange lipides/LOS/OG est ensuite dialysé contre du tampon Tris 10 mM pour éliminer l'OG et former les liposomes.

### Protocole

On réalise une préparation lipidique en chloroforme des lipides que l'on va utiliser pour la fabrication des liposomes. Un film sec est obtenu par évaporation complète du chloroforme.

Un film sec de 1,2 dioleyl-*sn*-glycero-3-ethylphosphocholine (EDOPC ou ethyl-DOPC) et de 1,2-dioleoyl-*sn*-glycero-3-phosphoethanolamine (DOPE) dans un rapport molaire EDOPC : DOPE de 3 pour 2 est obtenu en mélangeant 12.633 mL d'une solution de EDOPC (Avanti Polar Lipids ref 890704) à 20 mg/ml en chloroforme et 7.367 mL d'une solution de DOPE (Avanti Polar Lipids ref 850725) à 20 mg/ml en chloroforme et en évaporant le chloroforme jusqu'à disparition complète.

Le film sec est repris par 30 mL de tampon Tris 10 mM pH 7.0 pour obtenir une suspension contenant 13.333 mg de lipides /ml (8.42 mg/ml d'EDOPC et 4.91 mg/ml de DOPE). La suspension est agitée 1 hr à température ambiante puis soniquée 5 min dans un bain.

Puis on ajoute toujours sous agitation, 3.333 ml d'une solution stérile d'octyl β-D-glucopyranoside (OG) (Sigma-Aldrich ref 08001) 1 M en tampon Tris 10 mM pH 7.0 pour obtenir une suspension limpide de lipides à 12 mg/ml, OG 100 mM, tampon Tris 10 mM. On poursuit l'agitation 1 hr à température ambiante sur table d'agitation. Puis on filtre stérilement sur Millex HV 0.45 µm.

On prépare dans des conditions stériles, une composition en mettant en présence du LOS et des lipides dans un rapport molaire lipides : LOS de 250 (0.160 mg/mL de LOS, 9.412 mg/mL de lipides et 100 mM d'OG). 40 mL d'une telle composition résulte du mélange des les préparations suivantes :
2.005 mL de tampon Tris 10 mM pH 7.0 ; 0.223 mL d'OG 100 mM en Tris 10 mM ;
31.373 mL de la suspension EDOPC : DOPE de ratio molaire 3 : 2 à 12 mg/ml en OG 100 mM Tris 10 mM ; et 6.4 mL d'une suspension stérile de LOS à 1 mg/ml en OG 100 mM Tris 10 mM.

Après une heure d'agitation à température ambiante, la suspension est transférée stérilement dans 4 cassettes de dialyse stériles de 10 ml. Chaque cassette est dialysée 3 fois (24 hrs - 24 hrs - 72 hrs) contre 200 volumes de Tris 10 mM pH 7.0 soit 2 L.

On récupère les liposomes dans des conditions stériles. L'augmentation de volume après dialyse est environ de 30 %.

A cette préparation, on ajoute du merthiolate et du NaCl pour obtenir une préparation de liposomes en Tris 10 mM, NaCl 150 mM, pH 7.0, Merthiolate 0.001 % qui contient in fine environ 110 µg/ml de LOS et 7 mg/ml de lipides dont environ 4.5 mg/ml d'EDOPC et environ 2.5 mg/ml de DOPE (concentrations théoriques).

Les liposomes LOS sont conservés à +5°C.

### 3.2. Préparation des injectables

Les liposomes sont ajustés à la concentration requise en LOS (notamment pour les tests d'immunogénicité) en Tris 10 mM NaCl 150 mM pH 7.4. La concentration en merthiolate est maintenue à 0.001 %.

### 4. Préparation d'un mélange liposomes [LOS] + rTbpB

On mélange de la rTbpB en PBS (section B.1.3.) avec des liposomes [LOS] (section B.3.) dans un rapport poids : poids rTbpB : LOS égal à 1. Puis on ajuste en tampon Tris 10 mM, NaCl 150 mM, pH 7.4 pour obtenir une préparation dans laquelle chacun des composants (rTbpB et LOS) est concentré à 80 µg/ml. La concentration en merthiolate est maintenue à 0.001 %.

### 5. Production d'un endotoxoïde (LOS détoxifié par complexation à un peptide analogue de la polymixine B)

Cet endotoxoïde est préparé comme décrit dans la demande de brevet WO 06/108586. Brièvement, un volume d'une solution de LOS purifié à 1 mg/mL, stérilisée par filtration sur membrane de 0.22 µm, est mélangé à un volume d'une solution de peptide SAEP2-L2 à 1 mg/mL stérilisée par filtration sur membrane de 0.22 µm.

Le peptide SAEP2-L2 est un peptide de structure dimérique antiparallèle, de formule :

NH₂-Lys-Thr-Lys-Cys1-Lys-Phe-Leu-Leu-Leu-Cys2-COOH HOOC-Cys2-Leu-Leu-Leu-Phe-Lys-Cys1-Lys-Thr-Lys-NH₂

Un précipité se forme immédiatement. On mélange 5 min à température ambiante, puis on laisse reposer une nuit à 4 °C. Le précipité est récolté par centrifugation à 3,000 rpm pendant 10 min. Le culot est lavé 5 fois avec un volume d'eau stérile dépourvue de pyrogénicité, pH 7.2. Enfin, le culot est resuspendu en tampon Tris 10 mM NaCl 150 mM Tween 80, pH 7.4 pour obtenir une suspension à 1 mg/mL, calcul basé sur le poids humide du précipité. La suspension est conservée à 4°C.

### 6. Préparation d'un mélange endotoxoïde + rTbpB

On mélange de la rTbpB en PBS (obtenue tel que décrit dans la section B.1.3.) avec de l'endotoxoïde (section B.5.) dans un rapport poids : poids égal à 1. Puis on ajuste en tampon Tris 10 mM NaCl, 150 mM, Tween 80 0,05 % pour obtenir une préparation dans laquelle chacun des composants est concentré à 80 µg/ml.

### 7. Etude d'immunogénicité chez le lapin n°1

Les différentes formulations testées ont été fabriquées comme décrit dans l'une des précédentes sections.

### 7.1. Immunisations des lapins

Vingt quatre lapines NZ KBL (Charles River Lab.) âgées de 7 semaines sont réparties en 5 groupes test de quatre et en 2 groupes contrôle de deux.

Les lapines de chaque groupe reçoivent sous un volume de 0.5 ml reparti en 2 injections intramusculaires concomitantes dans les pattes, à J0, J21 et J42 :
Groupe A : 40 µg de liposomes [LOS chaîne α L6, PEA-3]
   en tampon Tris 10mM NACl 150 mM pH 7.4 ;
Groupe B : 40 µg de liposomes [LOS chaîne α L6, PEA-3] et 40 µg rTbpB M982
   en tampon Tris 10mM NACl 150 mM pH 7.4 ;
Groupe C : 40 µg de liposomes [LOS chaîne α L6, PEA-3, PEA-6]
   en tampon Tris 10mM NaCl 150 mM pH 7.4 ;
Groupe D : 40 µg de liposomes [LOS chaîne α L6, PEA-3, PEA-6] et 40 µg rTbpB M982
   en tampon Tris 10mM NACl 150 mM pH 7.4 ;
Groupe E : 40 µg de LOS chaîne α L6, PEA-3 sous forme d'endotoxoïde et 40 µg rTbpB M982
   en tampon Tris 10mM NACl 150 mM, Tween 0.5 % pH 7.0 ;
Groupe F (contrôle) : 40 µg rTbpB et des liposomes vides
   en tampon Tris 10mM NACl 150 mM pH 7.4 ; et
Groupe G (contrôle) : liposomes vides en tampon Tris 10mM NACl 150 mM pH 7.4.

Du sang des animaux est prélevé pour analyse à J0, J42 (avant la troisième injection) et à J56.

### 7.2. Dosage des anticorps anti-LOS par ELISA

Ce dosage est robotisé (robot Staccato, Caliper) selon le protocole suivant :
Les puits de plaques Dynex™ de 96 puits sont imprégnés avec pour chacun des groupes, 1 µg de LOS homologue en tampon PBS (phosphate buffer saline) 1X, pH 7.1 ° MgCl₂ 10mM, incubées 2 heures à 37°C puis une nuit à 4°C. On bloque les plaques en ajoutant dans les puits 150 µl de PBS contenant 0.05 % de Tween 20 et 1 % (poids/vol.) de lait écrémé en poudre (PBS-Tween-lait). Les plaques sont incubées 1 hr à 37°C.
Des dilutions en série de deux en deux des échantillons à tester sont réalisées en PBS - Tween 0.05 % - lait 1 %. Les plaques sont incubées 90 min à 37°C puis lavées 3 fois avec du PBS + Tween 20 à 0.05 %.

Un conjugué peroxydase - anti-IgG de souris ou de lapins en PBS-Tween-lait est ajouté dans les puits et les plaques sont incubées 90 min à 37°C. Les plaques sont lavées trois fois. On distribue 100 µl par puits d'une solution de TMB (3,3',5,5'-tétraméthylbenzidine, substrat de la peroxydase) prête à l'emploi. Les plaques sont incubées dans l'obscurité pendant 20 min à température ambiante. La réaction est stoppée en ajoutant 100 µl de HCl 1M par puits.

La densité optique est mesurée à 450-650 nm avec un lecteur automatique (Multiskan Ascent). En l'absence de standard, les titres anticorps sont déterminés comme étant la dilution réciproque donnant une densité optique de 1.0 sur une courbe Tendance (logiciel CodUnit). Le seuil de détection des anticorps est de 1.3 log₁₀ unité ELISA. Pour chaque titre inférieur à ce seuil, une valeur arbitraire de 1.3 log₁₀ est attribuée.

### 7.3. Mesure de l'activité bactéricide des IgGs purifiées à l'encontre de souches de N. meningitidis hétérologues à la souche C708

A partir des pools de sérums, les IgGs ont été purifiées par chromatographie d'affinité à l'aide de la colonne HiTrap rProtein A FF (GE Healthcare / Amersham Biosciences) selon les recommandations du fournisseur.

A partir des IgGs purifiées, on réalise des dilutions sérielles de raison 2 en PBS Dulbecco's gélatiné contenant des ions calcium et magnésium. Les dilutions sont réalisées en plaque 96 puits pour un volume final de 50 µL par puits.

L'activité bactéricide des IgGs purifiées a été testée à l'encontre des souches citées dans le tableau IV ci-après.

Vingt cinq µL d'une culture de *N. meningitidis* en phase exponentielle (4.10³ CFU/mL) en milieu BHI en absence d'agent chélateur du fer sous forme libre (pour éviter que la TbpB ne soit exprimée), ainsi que 25 µL de complément de bébé lapin au 1/1,5 sont ajoutés dans chaque puits. La plaque est incubée une heure à 37°C, sous agitation.

Cinquante µL du mélange de chaque puits sont ensuite déposés sur des boîtes de gélose Mueller-Hinton bioMérieux et incubées une nuit à 37°C sous 10 % de CO₂. On décompte le nombre de clones.

Les témoins sont au nombre de trois :
Bactéries + complément de bébé lapin, sans sérum à tester (témoin « complément ») ;
Bactéries + complément de bébé lapin inactivé, sans sérum à tester (témoin « germes ») ; et
Bactéries + complément de bébé lapin inactivé + sérum à tester (témoin sérum).

On exprime le titre bactéricide comme étant l'inverse de la dilution donnant 50 % de mort bactérienne par comparaison avec le témoin « complément ».

### 7.4. Résultats et Discussion

### ELISAs

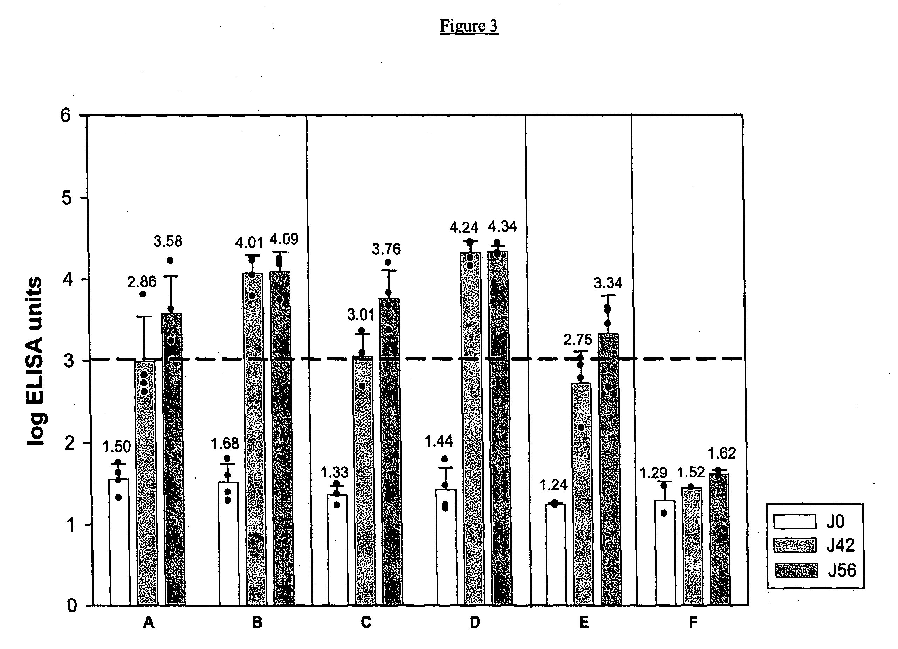

La figure 3 présente les titres ELISAs exprimés en log₁₀ des IgG anti-LOS des sérums de lapins des groupes A, B, C, D, E et F. En blanc, les titres des sérums avant immunisation ; en grisé, ceux des sérums prélevés à J42 après la deuxième immunisation ; et en foncé ceux des sérums prélevés à J56.

Les titres ELISAs aux temps J42 et J56 montrent que le LOS issu de chacune des souches C708 construites comme précédemment décrit est immunogène. Cette immunogénicité est accrue par la présence de la rTbpB lipidée pour chaque temps considéré (ce qui confirme le pouvoir d'adjuvant de cette lipoprotéine).

### Test de bactéricidie

L'activité bactéricide des IgGs purifiées est exprimée en «fold increase ». Le « fold : increase » est le rapport titre bactéricide des IgGs purifiées du groupe considéré : titre bactéricide du groupe contrôle négatif. Ainsi le taux de séroconversion en « fold increase » mesure l'accroissement du titre bactéricide. On considère que l'activité bactéricide est significative lorsqu'un facteur 8 est observé entre le groupe d'intérêt et le groupe témoin négatif correspondant (« fold increase » supérieur ou égal à x 8).

Les IgGs purifiées issues du groupe contrôle G présentent à l'encontre de toutes les souches testées un « fold increase » inférieur à x 4.

Les valeurs de « fold increase » obtenues avec les IgGs purifiées des groupes B et D à l'encontre de 18 souches sont reportées dans le tableau IV ci-après :

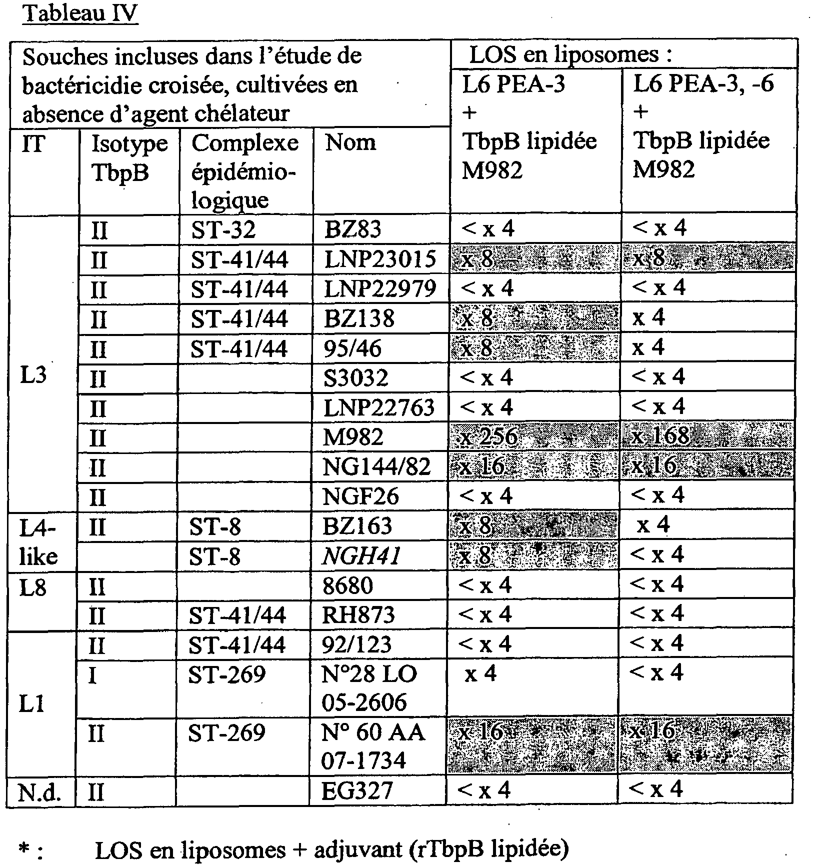

Les IgGs purifiées obtenues après immunisation avec la composition du groupe B (40 µg de liposomes [LOS chaîne α L6, PEA-3] et 40 µg rTbpB M982) ont été par la suite testées à l'encontre d'un plus grand nombre de souches. Les résultats sont reportés dans les tableaux V et VI ci-après.

### 8. Etude d'immunogénicité chez le lapin n°2

Les différentes formulations testées ont été fabriquées comme décrit dans l'une des précédentes sections.

### 8.1. Immunisations des lapins

Vingt quatre lapines NZ KBL (Charles River Lab.) âgées de 7 semaines sont réparties en 4 groupes test de quatre (groupes A à D) et en 4 groupes de deux (groupes E à H).

Les lapines de chaque groupe reçoivent sous un volume de 0.5 ml reparti en 2 injections intramusculaires concomitantes dans les pattes, à J0, J21 et J42 :
- Groupe A :: 40 µg de liposomes [LOS chaîne α L8, PEA-3, PEA-6] et 40 µg rTbpB M982,
en tampon Tris 10 mM NACl 150 mM pH 7.4 ;
- Groupe B :: 40 µg de liposomes [LOS chaîne α L8, PEA-3, PEA-6] et 40 µg rTbpB B16B6, en tampon Tris 10 mM NaCl 150 mM pH 7.4 ;
- Groupe C :: 40 µg de liposomes [LOS chaîne α L8, PEA-3] et 40 µg rTbpB M982, en tampon Tris 10 mM NACl 150 mM pH 7.4 ;
- Groupe D :: 40 µg de liposomes [LOS chaîne α L8, PEA-3, PEA-6] en tampon Tris 10 mM NACl 150 mM pH 7.4 ;
- Groupe E :: 40 µg rTbpB M982 et 40 µg de liposomes sans LOS en tampon Tris 10 mM NACl 150 mM, Tween 0.5 % pH 7.0 ;
- Groupe F :: 40 µg rTbpB B16B6 et 40 µg de liposomes sans LOS en tampon Tris 10 mM NaCl 150 mM, Tween 0.5 % pH 7.0 ;
- Groupe G :: 40 µg de liposomes [LOS chaîne α L8, PEA-3] en tampon Tris 10 mM NaCl 150 mM pH 7.4 ;
- Groupe H :: Tampon Tris 10 mM NACl 150 mM pH 7.4

Du sang des animaux est prélevé pour analyse à J0, J42 (avant la troisième injection) et à J56.

### 8.2. Mesure de l'activité bactéricide des IgGs purifiées à l'encontre de souches de N. meningitidis hétérologues à la souche C708

A partir des pools de sérums, les IgGs ont été purifiées par chromatographie d'affinité à l'aide de la colonne HiTrap rProtein A FF (GE Healthcare / Amersham Biosciences) selon les recommandations du fournisseur.

A partir des IgGs purifiées, on réalise des dilutions sérielles de raison 2 en PBS Dulbecco's gélatiné contenant des ions calcium et magnésium. Les dilutions sont réalisées en plaque de 96 puits pour un volume final de 50 µL par puits.

L'activité bactéricide des IgGs purifiées a été testée à l'encontre des souches citées dans le tableau V ci-après.

On réalise une culture des souches de *N. meningitidis* en milieu BHI complémenté ou non pendant 2 hrs 30 avc du Desféral 50µM (agent chélateur du fer sous forme libre, qui permet l'expression de la TbpB).

Vingt cinq µL de la culture en phase exponentielle (4.10³ CFU/ml) ainsi que 25 µL de complément de bébé lapin au 1/1,5 sont ajoutés dans chaque puits. La plaque est incubée une heure à 37°C, sous agitation.

Cinquante µL du mélange de chaque puits sont ensuite déposés sur des boîtes de gélose Mueller-Hinton bioMérieux et incubées une nuit à 37°C sous 10 % de CO₂. On décompte le nombre de clones.

Les témoins sont au nombre de trois :
Bactéries + complément de bébé lapin, sans sérum à tester (témoin « complément ») ;
Bactéries + complément de bébé lapin inactivé, sans sérum à tester (témoin « germes ») ; et
Bactéries + complément de bébé lapin inactivé + sérum à tester (témoin sérum).

On exprime le titre bactéricide comme étant l'inverse de la dilution donnant 50 % de mort bactérienne par comparaison avec le témoin « complément ».

### 8.3. Résultats et Discussion

### Test de bactéricidie

34 souches de *N. meningitidis* ont été testées en bactéricidie croisée. Leurs noms apparaissent dans le tableau V ci-dessous. Les résultats sont exprimés en « fold increase » selon la méthodologie de calcul décrite dans la section B.7.4. ci-dessus.

Comme attendu, les IgGs purifiées issues du groupe d'immunisation contrôle négatif ne présentent d'activité bactéricide à l'encontre d'aucune des souches.

Les IgGs purifiées issues des groupes d'immunisation D et G (pas d'adjuvantation du LOS avec une TbpB) font preuve d'une bactéricidie croisée de moindre intérêt. Par souci de simplicité, on ne présente dans le tableau V ci-après, que les résultats de bactéricidie exprimés en « fold increase » obtenus avec les IgGs purifiées des groupes A, B, C, E et F de l'étude n°2 avec les résultats obtenus avec les IgGs purifiées du groupe B de la première étude.

Le tableau VI présente les résultats de bactéricidie exprimés en « fold increase », des IgGs purifiées issues du groupe d'immunisation B de la première étude et du groupe A de la deuxième étude vis-à-vis de 22 souches cultivées en présence / absence de Desféral.

Le tableau VII présente pour diverses compositions vaccinales, le pourcentage de protection déduit des études de bactéricidie croisée incluant 34 souches cultivées en présence de Desféral.

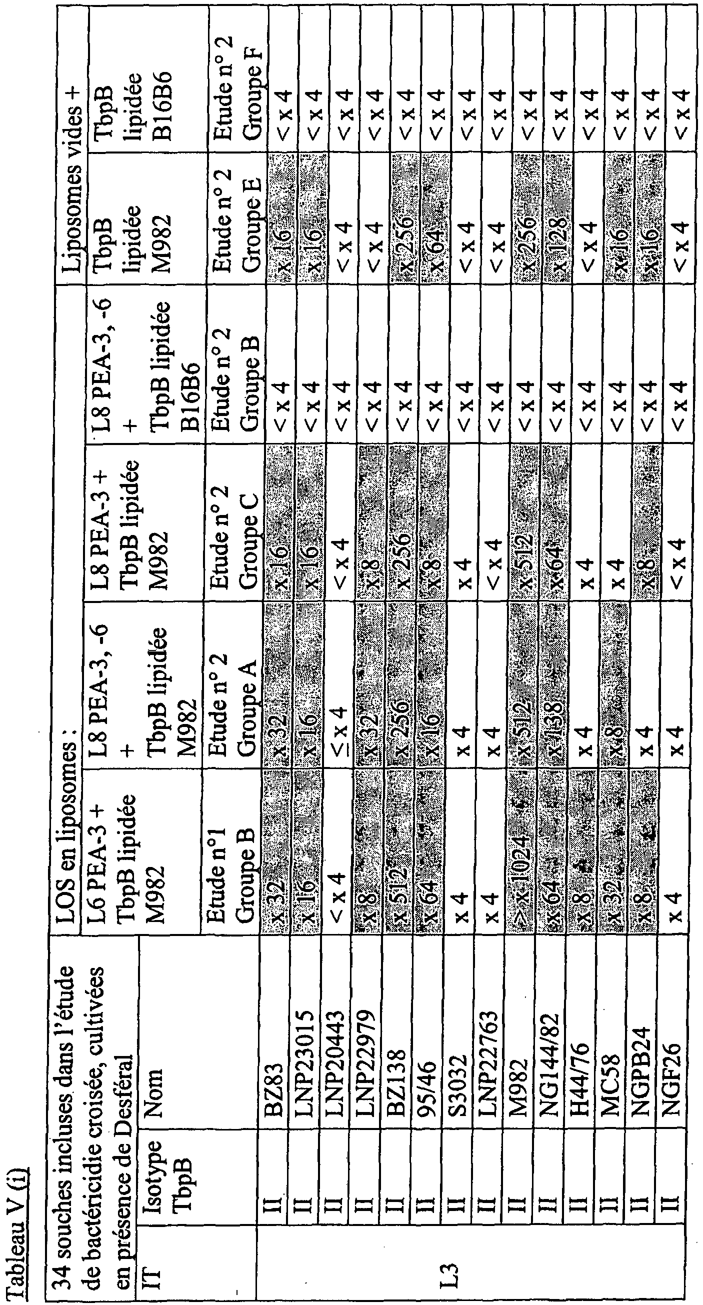

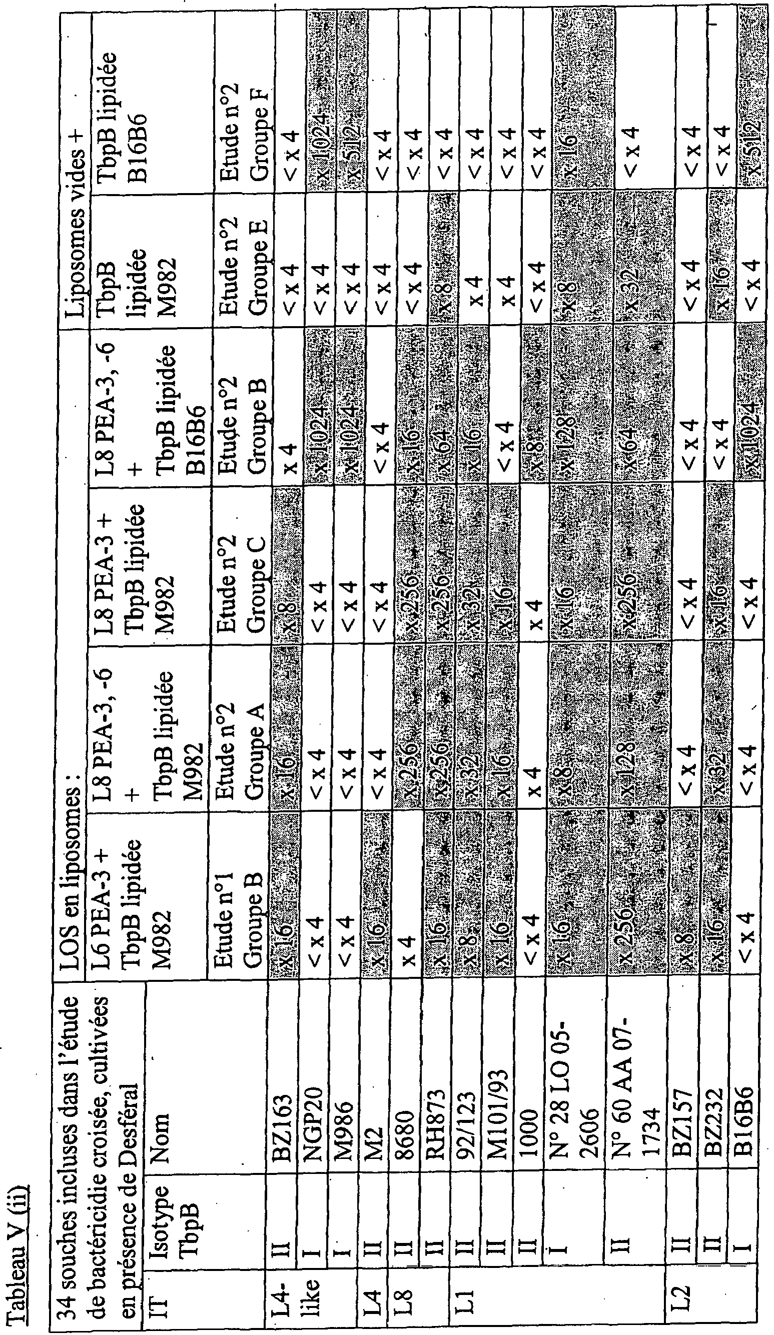

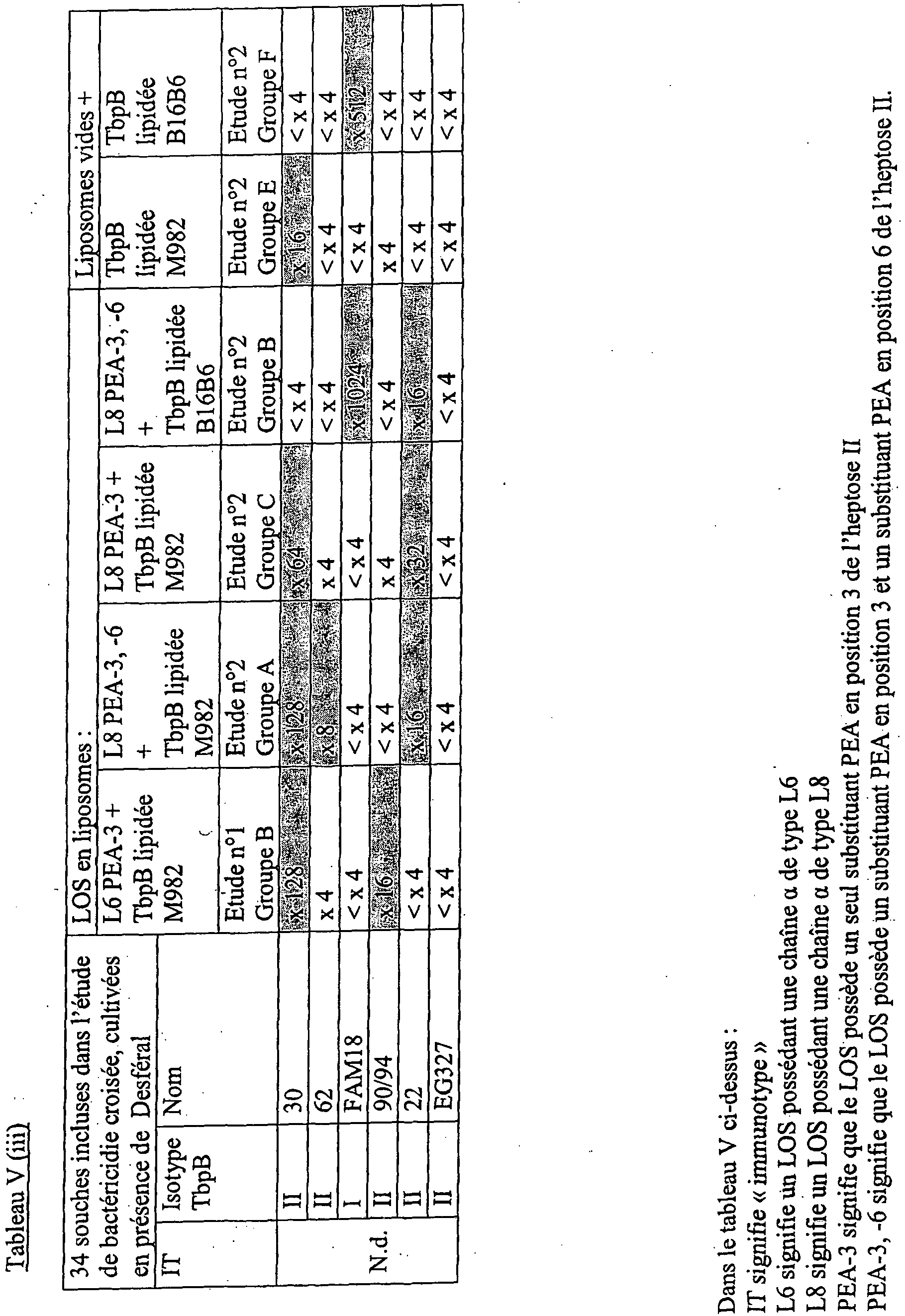

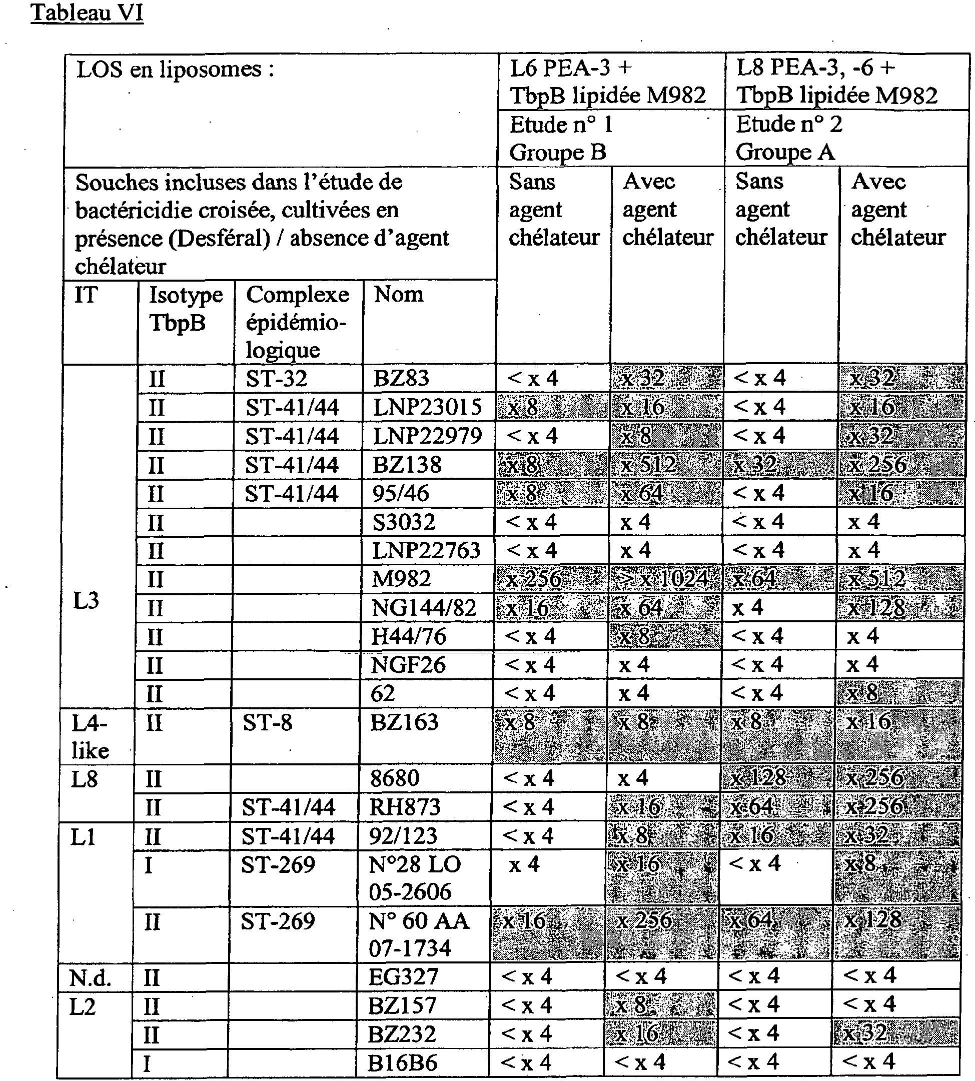

**Tableau VII**

| Compositions vaccinales | % de protection déduit des études de bactéricidie croisée incluant 34 souches cultivées en présence de Desféral |
|---|---|
| L6 PEA 03 + TbpB, M982 | 61.8 % |
| L8 PEA 03, 06 + TbpB, M982 | 55.9 % |
| L6 PEA 03 + L8 PEA 03, 06 + TbpB M982 | 70.6 % |
| L8 PEA 03 + TbpB M982 | 52.9 % |
| L6 PEA 03 + L8 PEA 03 + TbpB M982 | 67.6 % |
| L8 PEA 03, 06 + TbpB B16B6 | 32 % |
| L6 PEA 03 + TbpB M982 + TbpB B16B6 | 73.5 % |
| L8 PEA 03, 06 + TbpB, M982 + TbpB B16B6 | 58.8-67.6 % |
| L6 PEA 03 + L8 PEA 03, 06 + TbpB M982 + TbpB B16B6 | 82.4 % |
| L8 PEA 03 + TbpB M982 + TbpB B16B6 | 64.7 % |

## Revendications

1. Un procédé de fabrication d'une souche de *Neisseria meningitidis* possédant un lipooligosaccharide (LOS) constitué notamment d'un lipide A, d'un core interne, d'une chaîne α de type L6, dans lequel le résidu heptose II du core interne porte en position O-3 et en position O-6 ou O-7, un substituant phosphoéthanolamine (PEA) ; procédé selon lequel on modifie une souche de *N. meningitidis* d'immunotype L6 de telle sorte qu'elle exprime le gène *lpt3.*

2. Un procédé selon la revendication 1, dans lequel la souche que l'on modifie est une souche de *N. meningitidis* d'immunotype L6, sérogroupe A.

3. Un procédé selon la revendication 2, dans lequel la souche que l'on modifie est la souche C708 et dans lequel la modification consiste à restaurer la fonctionnalité du gène *lpt3* de la souche C708 (CNCM-3942).

4. Un procédé de fabrication d'une souche de *Neisseria meningitidis* possédant un lipooligosaccharide (LOS) constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; procédé selon lequel on modifie une souche de *N. meningitidis* d'immunotype L6 de telle sorte qu'elle exprime le gène *lpt3* et qu'elle n'exprime plus le gène *lpt6.*

5. Un procédé selon la revendication 4, dans lequel la souche que l'on modifie est une souche de *N. meningitidis* d'immunotype L6, sérogroupe A.

6. Un procédé selon la revendication 5, dans lequel la souche que l'on modifie est la souche C708 (CNCM-3942) en restaurant la fonctionnalité du gène *lpt3* de la souche C708 ; et en désactivant le gène *lpt6.*

7. Une souche de *Neisseria meningitidis* qui possède un lipooligosaccharide (LOS) constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA).

8. Une souche de *Neisseria meningitidis* selon la revendication 7, qui est de sérogroupe A.

9. Une souche de *Neisseria meningitidis* selon la revendication 7 ou 8, qui possède un gène *msb*B actif.

10. Une souche de *Neisseria meningitidis* selon la revendication 7, 8 ou 9, qui possède un LOS notamment constitué d'un lipide A non-détoxifié.

11. Une souche de *Neisseria meningitidis* de sérogroupe A, qui possède un lipooligosaccharide (LOS) constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7.

12. Une souche de *Neisseria meningitidis* selon la revendication 11, qui possède un gène *msb*B actif.

13. Une souche de *Neisseria meningitidis* selon la revendication 11 ou 12, qui possède un LOS notamment constitué d'un lipide A non-détoxifié.

14. Un lipooligosaccharide (LOS) de *Neisseria meningitidis* constitué notamment d'un lipide A, d'un core interne, d'une chaîne α de type L6, dans lequel le résidu heptose II du core interne porte en position O-3 et en position O-6 ou O-7 un substituant phosphoéthanolamine (PEA).

15. Un lipooligosaccharide (LOS) de *Neisseria meningitidis* selon la revendication 14, constitué notamment d'un lipide A non-détoxifié.

16. Un lipooligosaccharide (LOS) de *Neisseria meningitidis,* constitué notamment d'un lipide A non-détoxifié, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7.

17. Un procédé de préparation d'un lipooligosaccharide (LOS) de *Neisseria meningitidis* constitué notamment d'un lipide A, d'un core interne, d'une chaîne α de type L6, dans lequel le résidu heptose II du core interne porte en position O-3 et en position O-6 ou O-7, un substituant phosphoéthanolamine (PEA) ; procédé selon lequel (i) on cultive une souche obtenue à partir du procédé selon l'une des revendications 1 à 3 ou une souche selon l'une des revendications 7 à 10 et (ii) on récolte le LOS à partir de la culture obtenue à l'étape (i).

18. Un procédé de préparation d'un lipooligosaccharide (LOS) de *Neisseria meningitidis* constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7 ; procédé selon lequel (i) on cultive une souche obtenue à partir du procédé selon l'une des revendications 4 à 6 ou une souche selon l'une des revendications 11 à 13 et (ii) on récolte le LOS à partir de la culture obtenue à l'étape (i).

19. Une composition pharmaceutique à l'encontre des infections à *Neisseria meningitidis* qui comprend un LOS selon la revendication 16 ou obtenu selon le procédé selon la revendication 18, constitué notamment d'un lipide A, d'un core interne, d'une chaîne α de type L6, dans lequel le résidu heptose II du core interne porte en position O-3 un substituant phosphoéthanolamine (PEA) et ne porte pas de substituant PEA en positions O-6 et O-7.

20. Une composition selon la revendication 19, qui comprend en outre un LOS selon la revendication 14 ou 15 ou obtenu selon le procédé selon la revendication 17, constitué notamment d'un lipide A, d'un core interne, d'une chaîne α de type L6, dans lequel le résidu heptose II du core interne porte en position O-3 et en position O-6 ou O-7, un substituant phosphoéthanolamine (PEA).

21. Une composition selon la revendication 19 ou 20, qui comprend en outre un LOS de *N. meningitidis* constitué notamment d'un lipide A, d'un core interne, d'une chaîne α de type L6 ou L8, dans lequel le résidu heptose II du core interne porte en position O-6 ou O-7 un substituant phosphoéthanolamine (PEA) et ne porte pas de substituant PEA en position O-3.

22. Une composition pharmaceutique à l'encontre des infections à *Neisseria meningitidis* qui comprend un LOS selon la revendication 14 ou 15 ou obtenu selon le procédé selon la revendication 17 ; constitué notamment d'un lipide A, d'un core interne, d'une chaîne α de type L6, dans lequel le résidu heptose II du core interne porte en position O-3 et en position O-6 ou O-7, un substituant phosphoéthanolamine (PEA).

23. Une composition selon la revendication 19 ou 22, qui comprend en outre un LOS constitué notamment d'un lipide A, d'un core interne, d'une chaîne α de type L8, dans lequel le résidu heptose II du core interne porte en position O-3 un substituant phosphoéthanolamine (PEA) et ne porte pas de substituant PEA en positions O-6 et O-7.

24. Une composition selon la revendication 19 ou 22, qui comprend en outre un LOS constitué notamment d'un lipide A, d'un core interne, d'une chaîne α de type L8, dans lequel le résidu heptose II du core interne porte en position O-3 et en position O-6 ou O-7 un substituant phosphoéthanolamine (PEA).

25. Une composition selon l'une des revendications 19 à 24, dans laquelle le LOS est formulé en liposomes.

26. Une composition selon l'une des revendications 19 à 25, qui comprend en outre la sous-unité B du récepteur de la transferrine humaine de *Neisseria meningitidis* (TbpB lipidée) sous forme lipidée ou un fragment N-terminal lipidé de cette dernière.

27. Une composition selon la revendication 26, qui comprend (i) la sous-unité B du récepteur de la transferrine humaine de *Neisseria meningitidis* (TbpB lipidée) sous forme lipidée, d'isotype I ou un fragment N-terminal lipidé de cette dernière et (ii) la sous-unité B du récepteur de la transferrine humaine de *Neisseria meningitidis* (TbpB lipidée) sous forme lipidée, d'isotype II ou un fragment N-terminal lipidé de cette dernière.

## Patentansprüche

1. Verfahren zur Herstellung eines *Neisseria* meningitidis-Stamms mit einem Lipooligosaccharid (LOS), bestehend insbesondere aus einem Lipid A, aus einem inneren Kern, aus einer α-Kette des L6-Typs, worin der Heptoserest II des inneren Kerns in O-3-Position und in O-6- oder O-7-Position einen Phosphoethanolamin(PEA)-Substituenten trägt; wobei man bei dem Verfahren einen *N*. *meningitidis-*Stamm des L6-Immuntyps so modifiziert, dass er das *lpt*3-Gen exprimiert.

2. Verfahren nach Anspruch 1, wobei es sich bei dem Stamm, der modifiziert wird, um einen *N*. *meningitidis*-Stamm des Immuntyps L6, Serogruppe A, handelt.

3. Verfahren nach Anspruch 2, wobei es sich bei dem Stamm, der modifiziert wird, um den Stamm C708 handelt und wobei die Modifikation darin besteht, die Funktionalität des *lpt*3-Gens vom Stamm C708 (CNCM-3942) wiederherzustellen.

4. Verfahren zur Herstellung eines *Neisseria* meningitidis-Stamms mit einem Lipooligosaccharid (LOS), bestehend insbesondere aus einem Lipid A, aus einem inneren Kern, aus einer α-Kette des L6-Typs, worin der Heptoserest II des inneren Kerns in O-3-Position einen Phosphoethanolamin(PEA)-Substituenten trägt und keinen PEA-Substituenten in der O-6- und O-7-Position trägt; wobei man bei dem Verfahren einen *N*. *meningitidis-*Stamm des Immuntyps L6 so modifiziert, dass er das *lpt*3-Gen exprimiert und nicht mehr das *lpt*6-Gen exprimiert.

5. Verfahren nach Anspruch 4, wobei es sich bei dem Stamm, der modifiziert wird, um einen *N*. *meningitidis-*Stamm des Immuntyps L6, Serogruppe A, handelt.

6. Verfahren nach Anspruch 5, wobei es sich bei dem Stamm, der modifiziert wird, um den Stamm C708 (CNCM-3942) unter Wiederherstellung der Funktionsfähigkeit des *lpt*3-Gens von Stamm C708 und unter Deaktivierung des *lpt*6-Gens handelt.

7. *Neisseria-meningitidis-Stamm* mit einem Lipooligosaccharid (LOS), bestehend insbesondere aus einem Lipid A, aus einem inneren Kern, aus einer α-Kette des L6-Typs, worin der Heptoserest II des inneren Kerns in O-3-Position und in O-6-oder O-7-Position einen Phosphoethanolamin(PEA)-Substituenten trägt.

8. *Neisseria-meningitidis-*Stamm nach Anspruch 7, der zur Serogruppe A gehört.

9. *Neisseria-meningitidis-Stamm* nach Anspruch 7 oder 8, der über ein aktives msbB-Gen verfügt.

10. *Neisseria-meningitidis-Stamm* nach Anspruch 7, 8 oder 9, der über ein LOS, das insbesondere aus einem nichtdetoxifizierten Lipid A besteht, verfügt.

11. *Neisseria-meningitidis-Stamm* der Serogruppe A mit einem Lipooligosaccharid (LOS), bestehend insbesondere aus einem Lipid A, aus einem inneren Kern, aus einer α-Kette des L6-Typs, worin der Heptoserest II des inneren Kerns in O-3-Position einen Phosphoethanolamin(PEA)-Substituenten trägt und keinen PEA-Substituenten in O-6- und 0-7-Position trägt.

12. *Neisseria-meningitidis-*Stamm nach Anspruch 11, der über ein aktives *msb*B-Gen verfügt.

13. *Neisseria-meningitidis-*Stamm nach Anspruch 11 oder 12, der über ein LOS, das insbesondere aus einem nichtdetoxifizierten Lipid A besteht, verfügt.

14. Lipooligosaccharid (LOS) aus *Neisseria meningitidis,* bestehend insbesondere aus einem Lipid A, einem inneren Kern, einer α-Kette des L6-Typs, worin der Heptoserest II des inneren Kerns in O-3-Position und in O-6- oder O-7-Position einen Phosphoethanolamin(PEA)-Substituenten trägt.

15. Lipooligosaccharid (LOS) aus *Neisseria meningitidis* nach Anspruch 14, der insbesondere aus einem nichtdetoxifizierten Lipid A besteht.

16. Lipooligosaccharid (LOS) aus *Neisseria meningitidis,* bestehend insbesondere aus einem nichtdetoxifizierten Lipid A, einem inneren Kern, einer α-Kette des L6-Typs, worin der Heptoserest II des inneren Kerns in O-3-Position einen Phosphoethanolamin(PEA)-Substituenten trägt und keinen PEA-Substituenten in O-6- und O-7-Position trägt.

17. Verfahren zur Herstellung eines Lipooligosaccharids (LOS) aus *Neisseria meningitidis,* bestehend insbesondere aus einem Lipid A, einem inneren Kern, einer α-Kette des L6-Typs, worin der Heptoserest II des inneren Kerns in O-3-Position und in O-6- oder O-7-Position einen Phosphoethanolamin(PEA)-Substituenten trägt; wobei man bei dem Verfahren (i) einen ausgehend von dem Verfahren nach einem der Ansprüche 1 bis 3 erhaltenen Stamm oder einen Stamm nach einem der Ansprüche 7 bis 10 kultiviert und (ii) das LOS aus der in Schritt (i) erhaltenen Kultur erntet.

18. Verfahren zur Herstellung eines Lipooligosaccharids (LOS) aus *Neisseria meningitidis,* bestehend insbesondere aus einem Lipid A, einem inneren Kern, einer α-Kette des L6-Typs, worin der Heptoserest II des inneren Kerns in O-3-Position einen Phosphoethanolamin(PEA)-Substituenten trägt und keinen PEA-Substituenten - in O-6- und O-7- Position trägt; wobei man bei dem Verfahren (i) einen ausgehend von dem Verfahren nach einem der Ansprüche 4 bis 6 erhaltenen Stamm oder einen Stamm nach einem der Ansprüche 11 bis 13 kultiviert und (ii) das LOS aus der in Schritt (i) erhaltenen Kultur erntet.

19. Pharmazeutische Zusammensetzung gegen *Neisseria meningitidis-*Infektionen, die ein LOS nach Anspruch 16 oder ein nach dem Verfahren nach Anspruch 18 erhaltenes LOS, bestehend insbesondere aus einem Lipid A, einem inneren Kern, einer α-Kette des L6-Typs, worin der Heptoserest II des inneren Kerns in O-3-Position einen Phosphoethanolamin(PEA)-Substituenten trägt und keinen PEA-Substituenten in O-6- und O-7-Position trägt, umfasst.

20. Zusammensetzung nach Anspruch 19, die weiterhin ein LOS nach Anspruch 14 oder 15 oder ein nach dem Verfahren nach Anspruch 17 erhaltenes LOS, bestehend insbesondere aus einem Lipid A, einem inneren Kern, einer α-Kette des L6-Typs, worin der Heptoserest II des inneren Kerns in O-3-Position und in O-6- oder O-7-Position einen Phosphoethanolamin(PEA)-Substituenten trägt, umfasst.

21. Zusammensetzung nach Anspruch 19 oder 20, die weiterhin ein LOS aus *N. meningitidis,* bestehend insbesondere aus einem Lipid A, einem inneren Kern, einer α-Kette des L6- oder L8-Typs, worin der Heptoserest II des inneren Kerns in O-6- oder O-7-Position einen Phosphoethanolamin(PEA)-Substituenten trägt und keinen PEA-Substituenten in O-3-Position trägt, umfasst.

22. Pharmazeutische Zusammensetzung gegen *Neisseria meningitidis*-Infektionen, die ein LOS nach Anspruch 14 oder 15 oder ein nach dem Verfahren nach Anspruch 17 erhaltenes LOS, bestehend insbesondere aus einem Lipid A, einem inneren Kern, einer α-Kette des L6-Typs, worin der Heptoserest II des inneren Kerns in O-3-Position und in O-6- oder O-7-Position einen Phosphoethanolamin(PEA)-Substituenten trägt, umfasst.

23. Zusammensetzung nach Anspruch 19 oder 22, die weiterhin ein LOS, bestehend insbesondere aus einem Lipid A, einem inneren Kern, einer α-Kette des L8-Typs, worin der Heptoserest II des inneren Kerns in O-3-Position einen Phosphoethanolamin(PEA)-Substituenten trägt und keinen PEA-Substituenten in O-6- und O-7-Position trägt, umfasst.

24. Zusammensetzung nach Anspruch 19 oder 22, die weiterhin ein LOS, bestehend insbesondere aus einem Lipid A, einem inneren Kern, einer α-Kette des L8-Typs, worin der Heptoserest II des inneren Kerns in O-3-Position und in O-6- oder 0-7-Position einen Phosphoethanolamin(PEA)-Substituenten trägt, umfasst.

25. Zusammensetzung nach einem der Ansprüche 19 bis 24, worin das LOS mit Liposomen formuliert ist.

26. Zusammensetzung nach einem der Ansprüche 19 bis 25, der weiterhin die Untereinheit B des Humantransferrinrezeptors aus *Neisseria meningitidis* (lipidierte TbpB) in lipidierter Form oder ein lipidiertes N-terminales Fragment davon umfasst.

27. Zusammensetzung nach Anspruch 26, die (i) die Untereinheit B des Humantransferrinrezeptors aus *Neisseria meningitidis* (lipidierte TbpB) in lipidierter Form des Isotyps I oder ein lipidiertes N-terminales Fragment davon und (ii) die Untereinheit B des Humantransferrinrezeptors aus *Neisseria meningitidis* (lipidierte TbpB) in lipidierter Form des Isotyps II oder ein lipidiertes N-terminales Fragment davon, umfasst.

## Claims

1. Process for manufacturing a strain of *Neisseria meningitidis* bearing a lipooligosaccharide (LOS) formed especially from a lipid A, an inner core, an α chain of L6 type, in which the heptose II residue of the inner core bears in position O-3 and in position O-6 or O-7 a phosphoethanolamine (PEA) substituent; according to which process an *N*. *meningitidis* strain of immunotype L6 is modified such that it expresses the *lpt3* gene.

2. Process according to Claim 1, in which the strain that is modified is an N. *meningitidis* strain of immunotype L6, serogroup A.

3. Process according to Claim 2, in which the strain that is modified is the strain C708 and in which the modification consists in restoring the functionality of the *lpt*3 gene of the strain C708 (CNCM-3942).

4. Process for manufacturing a strain of *Neisseria meningitidis* bearing a lipooligosaccharide (LOS) formed especially from a lipid A, an inner core, an α chain of L6 type, in which the heptose II residue of the inner core bears in position O-3 a phosphoethanolamine (PEA) substituent and does not bear a PEA substituent in positions O-6 and O-7; according to which process an *N*. *meningitidis* strain of immunotype L6 is modified such that it expresses the *lpt*3 gene and such that it no longer expresses the *lpt*6 gene.

5. Process according to Claim 4, in which the strain that is modified is an *N*. *meningitidis* strain of immunotype L6, serogroup A.

6. Process according to Claim 5, in which the strain that is modified is the strain C708 (CNCM-3942) by restoring the functionality of the *lpt3* gene of the strain C708; and by deactivating the *lpt*6 gene.

7. Strain of *Neisseria meningitidis* bearing a lipooligosaccharide (LOS) formed especially from a lipid A, an inner core, an α chain of L6 type, in which the heptose II residue of the inner core bears in position O-3 and in position O-6 or O-7 a phosphoethanolamine (PEA) substituent.

8. Strain of *Neisseria meningitidis* according to Claim 7, which is of serogroup A.

9. Strain of *Neisseria meningitidis* according to Claim 7 or 8, which bears an active *msb*B gene.

10. Strain of *Neisseria meningitidis* according to Claim 7, 8 or 9, which bears an LOS formed especially from a non-detoxified lipid A.

11. Strain of *Neisseria meningitidis* of serogroup A, which bears a lipooligosaccharide (LOS) formed especially from a lipid A, an inner core, an α chain of L6 type, in which the heptose II residue of the inner core bears in position O-3 a phosphoethanolamine (PEA) substituent and does not bear a PEA substituent in positions O-6 and O-7.

12. Strain of *Neisseria meningitidis* according to Claim 11, which bears an active *msb*B gene.

13. Strain of *Neisseria meningitidis* according to Claim 11 or 12, which bears an LOS formed especially from a non-detoxified lipid A.

14. Lipooligosaccharide (LOS) of *Neisseria meningitidis* formed especially from a lipid A, an inner core, an α chain of L6 type, in which the heptose II residue of the inner core bears in position O-3 and in position O-6 or O-7 a phosphoethanolamine (PEA) substituent.

15. Lipooligosaccharide (LOS) of *Neisseria meningitidis* according to Claim 14, formed especially of a non-detoxified lipid A.

16. Lipooligosaccharide (LOS) of *Neisseria meningitidis,* formed especially from a non-detoxified lipid A, an inner core, an α chain of L6 type, in which the heptose II residue of the inner core bears in position O-3 a phosphoethanolamine (PEA) substituent and does not bear any PEA substituent in positions O-6 and O-7.

17. Process for preparing a lipooligosaccharide (LOS) of *Neisseria meningitidis* formed especially from a lipid A, an inner core, an α chain of L6 type, in which the heptose II residue of the inner core bears in position O-3 and in position O-6 or O-7 a phosphoethanolamine (PEA) substituent; according to which process (i) a strain obtained using the process according to one of Claims 1 to 3 or a strain according to one of Claims 7 to 10 is cultured and (ii) the LOS is harvested from the culture obtained in step (i).

18. Process for preparing a lipooligosaccharide (LOS) of *Neisseria meningitidis* formed especially from a lipid A, an inner core, an α chain of L6 type, in which the heptose II residue of the inner core bears in position O-3 a phosphoethanolamine (PEA) substituent and does not bear a PEA substituent in positions O-6 and O-7; according to which process (i) a strain obtained using the process according to one of Claims 4 to 6 or a strain according to one of Claims 11 to 13 is cultured and (ii) the LOS is harvested from the culture obtained in step (i).

19. Pharmaceutical composition for combating infections caused by *Neisseria meningitidis* which comprises an LOS according to Claim 16 or obtained according to the process according to Claim 18 formed especially from a lipid A, an inner core, an α chain of L6 type, in which the heptose II residue of the inner core bears in position O-3 a phosphoethanolamine (PEA) substituent and does not bear any PEA substituent in positions O-6 and O-7.

20. Composition according to Claim 19, which additionally comprises an LOS according to Claim 14 or 15 or obtained according to the process according to Claim 17 formed especially from a lipid A, an inner core, an α chain of L6 type, in which the heptose II residue of the inner core bears in position O-3 and in position O-6 or O-7, a phosphoethanolamine (PEA) substituent.

21. Composition according to Claim 19 or 20, which also comprises an LOS of *N. meningitidis* formed especially from a lipid A, an inner core, an α chain of L6 or L8 type, in which the heptose II residue of the inner core bears in position O-6 or O-7 a phosphoethanolamine (PEA) substituent and does not bear a PEA substituent in position O-3.

22. Pharmaceutical composition for combating infections caused by *Neisseria meningitidis,* which comprises an LOS according to Claim 14 or 15 or obtained according to the process according to Claim 17; formed especially from a lipid A, an inner core, an α chain of L6 type, in which the heptose II residue of the inner core bears in position O-3 and in position O-6 or O-7 a phosphoethanolamine (PEA) substituent.

23. Composition according to Claim 19 or 22, which also comprises an LOS formed especially from a lipid A, an inner core, an α chain of L8 type, in which the heptose II residue of the inner core bears in position O-3 a phosphoethanolamine (PEA) substituent and does not bear a PEA substituent in positions O-6 and O-7.

24. Composition according to Claim 19 or 22, which also comprises an LOS formed especially from a lipid A, an inner core, an α chain of L8 type, in which the heptose II residue of the inner core bears in position O-3 and in position O-6 or O-7 a phosphoethanolamine (PEA) substituent.

25. Composition according to one of Claims 19 to 24, in which the LOS is formulated as liposomes.

26. Composition according to one of Claims 19 to 25, which also comprises the subunit B of the human transferrin receptor of *Neisseria meningitidis* (lipidated TbpB) in lipidated form or a lipidated N-terminal fragment thereof.

27. Composition according to Claim 26, which comprises (i) the subunit B of the human transferrin receptor of *Neisseria meningitidis* (lipidated TbpB) in lipidated form, of isotype I or a lipidated N-terminal fragment thereof, and (ii) the subunit B of the human transferrin receptor of *Neisseria meningitidis* (lipidated TbpB) in lipidated form, of isotype II, or a lipidated N-terminal fragment thereof.
